# EUROPEAN PATENT SPECIFICATION

(11) **EP 1 472 353 B1**
(45) Date of publication and mention of the grant of the patent: **01.02.2012**
(21) Application number: 03701402.4
(22) Date of filing: 11.02.2003
(51) Int. Cl.: C12N 15/31, C12N 15/62, C07K 14/315, A61K 39/09, C07K 16/12, G01N 33/53

(54) **GROUP B STREPTOCOCCUS ANTIGEN**
ANTIGEN VON STREPTOCOCCUS AUS DER B-GRUPPE
ANTIGENES DU STREPTOCOQUE DU GROUPE B

(30) Priority: 11.02.2002 US 354947 P
(43) Date of publication of application: 03.11.2004
(73) Proprietor: ID Biomedical Corporation, Laval, QC H7V 3S8 (CA)
(72) Inventor: RIOUX, Stéphane, Blainville, Quebec J7C 4T6 (CA); MARTIN, Denis, Sainte Thérèse, Québec J7E 4P5 (CA); HAMEL, Josée, Sillery, Quebec G1T 1N6 (CA); BRODEUR, Bernard, R., Sillery, Québec G1T 1N6 (CA)
(74) Representative: Lubienski, Michael John
(86) International application number: PCT/CA2003/000186
(87) International publication number: WO 2003/068813

(56) References cited:
- WO-A-01/32882
- WO-A-99/42588
- DATABASE SWALL [Online] EBI; 1 December 2001 (2001-12-01) "Truncated group B streptococcal surface immunogenic protein (SIP)" Database accession no. Q93GJ8 XP002247834
- BRODEUR B R ET AL: "Identification of group B streptococcal Sip protein, which elicits cross-protective immunity" INFECTION AND IMMUNITY, AMERICAN SOCIETY FOR MICROBIOLOGY. WASHINGTON, US, vol. 68, no. 10, October 2000 (2000-10), pages 5610-5618, XP002219117 ISSN: 0019-9567 cited in the application

## Description

### FIELD OF THE INVENTION

The present invention is related to polypeptides, epitopes and antibodies directed to these epitopes, more particularly to the Sip polypeptide of Group B streptococcus (GBS), also called Streptococcus Agalactiae which may be used to prevent, diagnose and/or treat streptococcal infection.

### BACKGROUND OF THE INVENTION

Streptococcus are gram (+) bacteria that are differentiated by group specific carbohydrate antigens A through O found on their cell surface. Streptococcus groups are further distinguished by type-specific capsular polysaccharide antigens. Several serotypes have been identified for the Group B streptococcus (GBS): Ia, Ib, II, III, IV, V, VI, VII and VIII. GBS also contains antigenic proteins known as "C-proteins" (alpha, beta, gamma and delta), some of which have been cloned.

Although GBS is a common component of the normal human vaginal and colonic flora this pathogen has long been recognized as a major cause of neonatal sepsis and meningitis, late-onset meningitis in infants, postpartum endometritis as well as mastitis in dairy herds. Expectant mothers exposed to GBS are at risk of postpartum infection and may transfer the infection to their baby as the child passes through the birth canal. Although the organism is sensitive to antibiotics, the high attack rate and rapid onset of sepsis in neonates and meningitis in infants results in high morbidity and mortality.

GBS infections in infants are restricted to very early infancy. Approximately 80% of infant infections occur in the first days of life, so-called early-onset disease. Late-onset infections occur in infants between 1 week and 2 to 3 months of age. Clinical syndromes of GBS disease in newborns include sepsis, meningitis, pneumonia, cellulitis, osteomyelitis, septic arthritis, endocarditis, epiglottis. In addition to acute illness due to GBS, which is itself costly, GBS infections in newborns can result in death, disability, and, in rare instances, recurrence of infection. Although the organism is sensitive to antibiotics, the high attack rate and rapid onset of sepsis in neonates and meningitis in infants results in high morbidity and mortality.

Among pregnant women, GBS causes clinical illness ranging from mild urinary tract inflection to life-threatening sepsis and meningitis, including also osteomyelitis, endocarditis, amniotis, endometritis, wound infections (postcesarean and postepisiotomy), cellulitis, fasciitis.

Among non-pregnant adults, the clinical presentations of invasive GBS disease most often take the form of primary bacteremia but also skin of soft tissue infection, pneumonia, urosepsis, endocarditis, peritonitis, meningitis, empyema. Skin of soft tissue infections include cellulitis, infected peripheral ulcers, osteomyelitis, septic arthritis and decubiti or wound infections. Among people at risk, there are debilitated hosts such as people with a chronic disease such as diabetes mellitus and cancer, or elderly people.

GBS infections can also occur in animals and cause mastitis in dairy herds.

To find a vaccine that will protect individuals from GBS infection, researchers have turned to the type-specific antigens. Unfortunately these polysaccharides have proven to be poorly immunogenic in humans and are restricted to the particular serotype from which the polysaccharide originates. Further, capsular polysaccharide antigens are unsuitable as a vaccine component for protection against GBS infection.

Others have focused on the C-protein beta antigen which demonstrated immunogenic properties in mice and rabbit models. This protein was found to be unsuitable as a human vaccine because of its undesirable property of interacting with high affinity and in a non-immunogenic manner with the Fc region of human IgA. The C-protein alpha antigens is rare in type III serotypes of GBS which is the serotype responsible for most GBS mediated conditions and is therefore of little use as a vaccine component.

PCT WO 99/42588 has been published February 17, 1999 entitled 'Group B streptococcus antigens' describing the polypeptide ID-42 which is claimed to be antigenic. This polypeptide is now known under the name Sip, for Surface immunogenic protein (Brodeur et al., 2000, Infect. Immun. 68:5610).

This polypeptide was found to be highly conserved and produced by every GBS examined to date, which included representative isolates of all serotypes (Brodeur et al., 2000, Infect. Immun. 68:5610). This. 53-kDa polypeptide is recognized by the human immune system. More importantly, immunization of adult mice with the Sip-polypeptide was shown to induce a strong specific antibody response and to confer protection against experimental infection with GBS strains representing serotypes Ia/c, Ib, II/R, III, V and VI (Brodeur et al.). It was also demonstrated that Sip-specific antibodies recognized their epitopes at the cell surfaces of different GBS strains, which included representatives of all nine serotypes (Rioux et al., 2001, Infect. Immun. 69:5162). In addition, it was recently reported that passive administration of rabbit anti-Sip serum to pregnant mice or immunization of female mice before pregnancy with purified recombinant Sip conferred protective immunity to their offsprings against GBS infection (Martin et al. Abstr. 101th Gen. Meet. Am. Soc. Microbiol. 2001).

Therefore there remains an unmet need for Group B Streptococcus polypeptides that may be used used to prevent, diagnose and/or treat Group B Streptococcus infection. Data describing the localization of surface-accessible regions on the Sip polypeptide of Group B Streptococcus are presented. Examples presenting the utilization of these surface-accessible regions for vaccine development are also presented.

### SUMMARY OF THE INVENTION

According to one aspect, the present invention provides an isolated polynucleotide encoding a polypeptide consisting of an amino acid sequence at least 85% identical over the full length of the amino acid sequence chosen from: SEQ ID NOS: 4, 6, 8, and 12 wherein the encoded polypeptide retains the ability to raise antibodies having binding specificity for Group B Streptococcus. Also disclosed herein is an isolated polynucleotide encoding a polypeptide having at least 70% identity to a second polypeptide comprising a sequence chosen from SEQ ID Nos : 1 2, 4, 6, 8, 10, 12, 14, 16, 18, 20 or fragments or analogs thereof.

In other aspects, there are provided polypeptides encoded by polynucleotides of the invention in so for as encompassed by the appendant claims, vectors comprising polynucleotides of the invention operably linked to an expression control region, as well as host cells transfected with said vectors and processes for producing polypeptides comprising culturing said host cells under conditions suitable for expression.

In a further aspect the present invention provides a method for detection of an antibody specific to Group B Streptococcus antigen in a biological sample previously obtained from a host, comprising:
(a) incubating one or more of the polypeptides according to any one of Claims 11 to 16 with the biological sample to form a mixture; and
(b) detecting specifically bound polypeptide in the mixture which indicates the presence of an antibody specific to the Group B Streptococcus antigen in the sample.
In a further aspect the present invention provides a kit comprising the polypeptide according to any one of Claims 11 to 15 for detection or diagnosis of Group B Streptococcus infection.

### BRIEF DESCRIPTION OF THE DRAWINGS

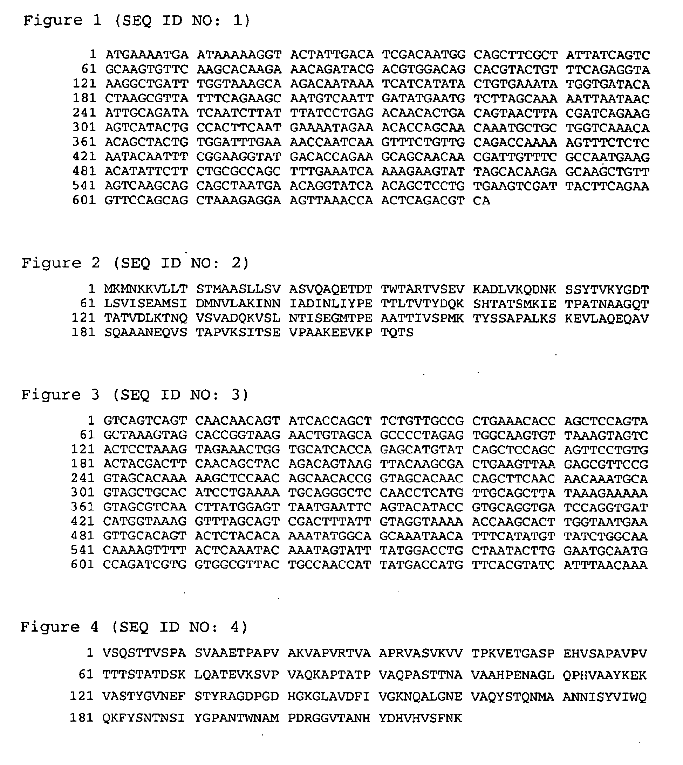

Figure 1 represents the DNA sequence of Δsip-1 gene from serotype Ia/c Group B streptococcus strain C388/90; (SEQ ID NO: 1).
Figure 2 represents the amino acid sequence of ΔSip-1 polypeptide from serotype I a/c Group B streptococcus strain C388/90; (SEQ ID NO: 2).
Figure 3 represents the DNA sequence- of Δsip-2 gene from serotype Ia/c Group B streptococcus strain C388/90; (SEQ ID NO: 3).
Figure 4 represents the amino acid sequence of ΔSip-2 polypeptide from serotype I a/c Group B streptococcus strain C388/90; (SEQ ID NO: 4).

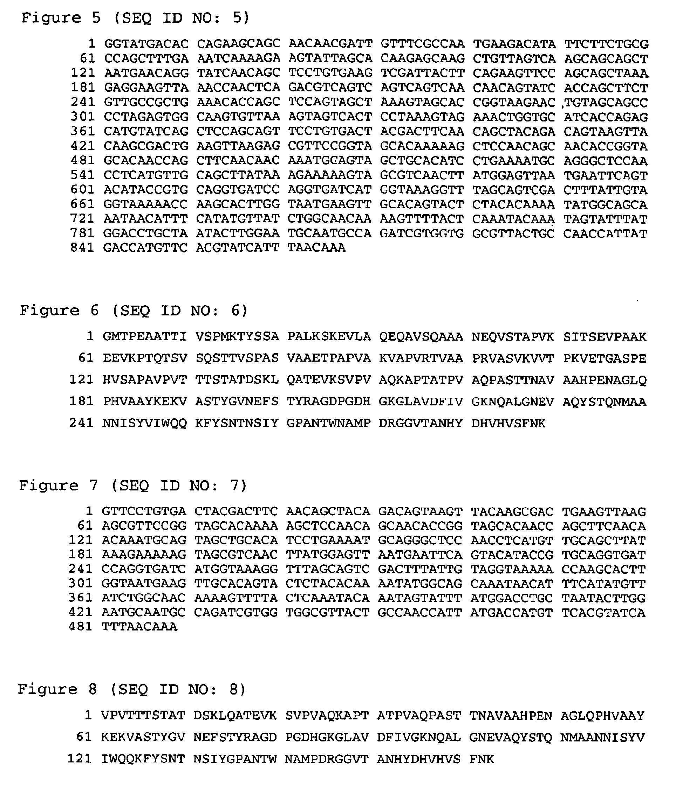

Figure 5 represents the DNA sequence of Δsip-3 gene from serotype Ia/c Group B streptococcus strain C388/90; (SEQ ID NO: 5).
Figure 6 represents the amino acid sequence of ΔSip-3 polypeptide from serotype I a/c Group B streptococcus strain C388/90; (SEQ ID NO: 6).
Figure 7 represents the DNA sequence of Δsip-4 gene from serotype Ia/c Group B streptococcus strain C388/90; (SEQ ID NO: 7).
Figure 8 represents the amino acid sequence of ΔSip-4 polypeptide from serotype I a/c Group B streptococcus strain C388/90; (SEQ ID NO: 8).

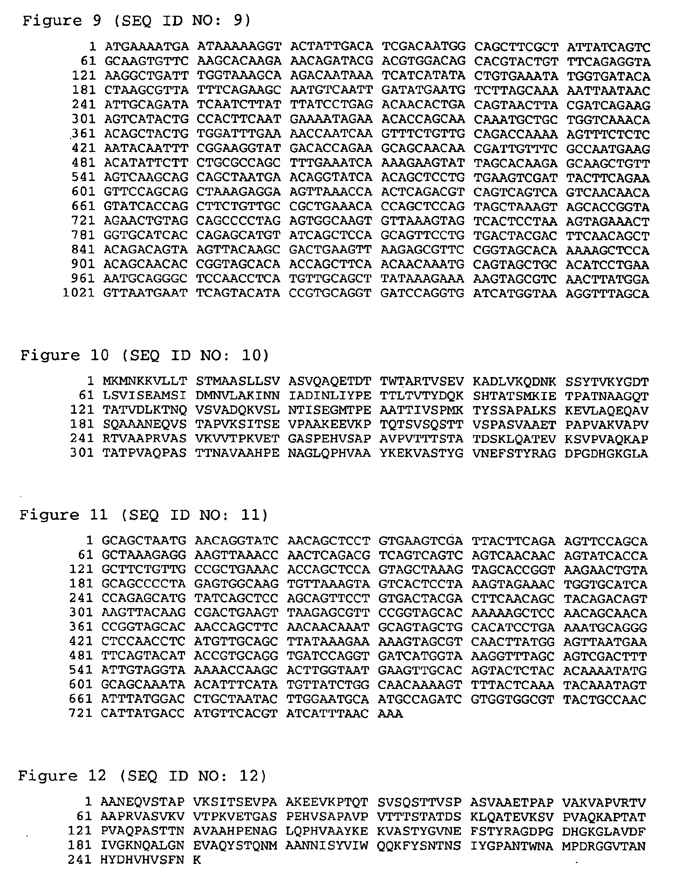

Figure 9 represents the DNA sequence of Δsip-5 gene from serotype Ia/c Group B streptococcus strain C388/90; (SEQ ID NO: 9).
Figure 10 represents the amino acid sequence of ΔSip-5 polypeptide from serotype I a/c Group B streptococcus strain C388/90; (SEQ ID NO: 10).
Figure 11 represents the DNA sequence of Δsip-6 gene from serotype Ia/c Group B streptococcus strain C388/90; (SEQ ID NO: 11).
Figure 12 represents the amino acid sequence of ΔSip-6 polypeptide from serotype I a/c Group B streptococcus strain C388/90; (SEQ ID NO: 12).

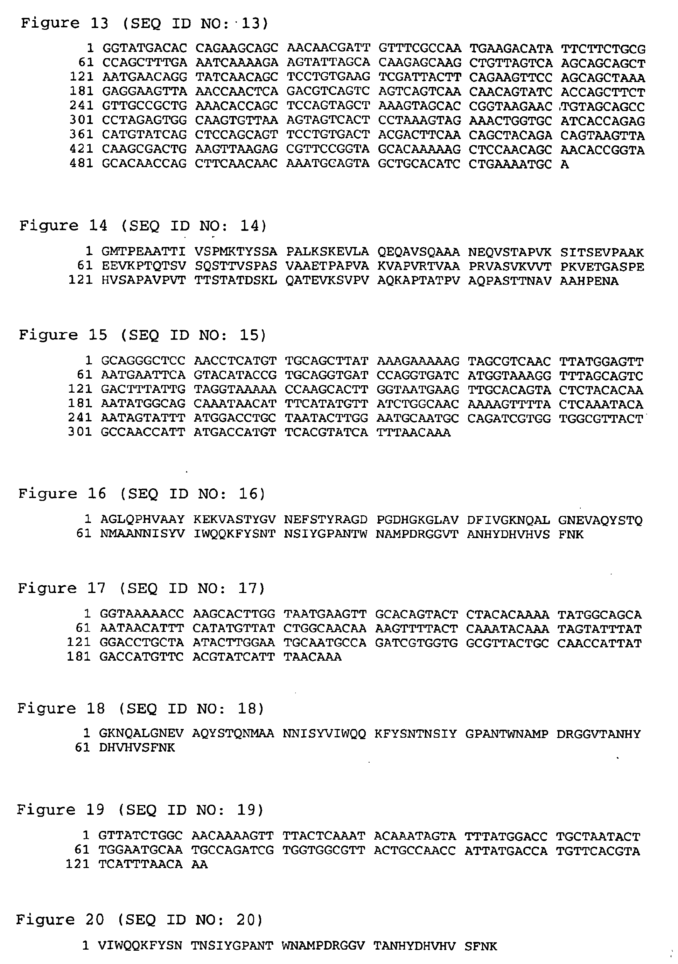

Figure 13 represents the DNA sequence of Δsip-7 gene from serotype Ia/c Group B streptococcus strain C388/90; (SEQ ID NO: 13).
Figure 14 represents the amino acid sequence of ΔSip-7 polypeptide from serotype I a/c Group B streptococcus strain C388/90; (SEQ ID NO: 14).
Figure 15 represents the DNA sequence of Δsip-8 gene from serotype Ia/c Group B streptococcus strain C388/90; (SEQ ID NO: 15).
Figure 16 represents the amino acid sequence of Δip-8 polypeptide from serotype I a/c Group B streptococcus strain C388/90; (SEQ ID NO: 16).
Figure 17 represents the DNA sequence of Δsip-9 gene from serotype Ia/c Group B streptococcus strain C388/90; (SEQ ID NO: 17).
Figure 18 represents the amino acid sequence of ΔSip-9 polypeptide from serotype I a/c Group B streptococcus strain C388/90; (SEQ ID NO: 18).
Figure 19 represents the DNA sequence of Δsip-10 gene from serotype Ia/c Group B streptococcus strain C388/90; (SEQ ID NO: 19).
Figure 20 represents the amino acid sequence of ΔSip-10 polypeptide from serotype I a/c Group B streptococcus strain C388/90; (SEQ ID NO: 20).

### DETAILED DESCRIPTION OF THE INVENTION

The present invention provides purified and isolated polynucleotides, which encode Streptococcus polypeptides which may be used to prevent, diagnose and/or treat Streptococcus infection. The polynucleotides are as defined in the appendant claims.

Also disclosed herein are: an isolated polynucleotide encoding a polypeptide having at least 70% identity to a second polypeptide comprising a sequence chosen from SEQ ID NO: 2, 4, 6, 8, 10, 12, 14, 16, 18, 20 or fragments or analogs thereof ;
an isolated polynucleotide encoding a polypeptide having at least 80% identity to a second polypeptide comprising a sequence chosen from SEQ ID NO: 2, 4, 6, 8, 10, 12, 14, 16, 18, 20 or fragments or analogs thereof;
an isolated polynucleotide encoding a polypeptide having at least 95% identity to a second polypeptide comprising a sequence chosen from SEQ ID NO: 2, 4, 6, 8, 10, 12, 14, 16, 18, 20 or fragments or analogs thereof;
a polynucleotide encoding an epitope bearing portion of a polypeptide comprising a sequence chosen from: SEQ ID NOs: 2, 4, 6, 8, 10, 12, 14, 16, 18 or 20 or fragments or analogs or thereof;
an epitope bearing portions of a polypeptide comprising a sequence chosen from SEQ ID NOS: 2, 4, 6, 8, 10, 12, 14, 16, 18 or 20 or fragments or analogs or thereof;
an isolated polynucleotide encoding a polypeptide having at least 70% identity to a second polypeptide comprising a sequence chosen from SEQ ID NO: 2, 4, 6, 8, 10, 12, 14, 16, 18, 20;
an isolated polynucleotide encoding a polypeptide having at least 80% identity to a second polypeptide comprising a sequence chosen from SEQ ID NO: 2, 4, 6, 8, 10, 12, 14, 16, 18, 20;
an isolated polynucleotide encoding a polypeptide having at least 95% identity to a second polypeptide comprising a sequence chosen from SEQ ID NO: 2, 4, 6, 8, 10, 12, 14, 16, 18, 20;
a polynucleotide encoding an epitope bearing portion of a polypeptide comprising a sequence chosen from: SEQ ID NOs: 2, 4, 6, 8, 10, 12, 14, 16, 18 or 20;
an epitope bearing portions of a polypeptide comprising a sequence chosen from SEQ ID NOS: 2, 4, 6, 8, 10, 12, 14, 16, 18 or 20.

The present invention provides an isolated polypeptide consisting of an amino acid sequence at least 85% identical over the full length of the amino acid sequence chosen from: SEQ ID NOS: 4, 6, 8, and 12, wherein the polypeptide retains the ability to raise antibodies having binding specificity for Group B Streptococcus. The invention further provides the polypeptide of Claim 11 consisting of an amino acid sequence at least 90% identical over the full length of the amino acid sequence chosen from SEQ ID NOS: 4, 6, 8, and 12. The invention further provides the polypeptide of Claim 11 consisting of an amino acid sequence at least 95% identical over the full length of the amino acid sequence chosen from SEQ ID NOS: 4, 6, 8, and 12. The invention further provides the isolated polypeptide of Claim 11 consisting of an amino acid sequence at least 99% identical over the full length of the amino acid sequence chosen from SEQ ID NOS: 4, 6, 8, and 12. The invention further provides the isolated polypeptide of Claim 11 consisting of the amino acid sequence chosen from SEQ ID NOS:4, 6, 8, and 12.

The present disclosure provides an isolated polypeptide comprising a polypeptide chosen from:
(a) a polypeptide having at least 70% identity to a second polypeptide comprising a sequence chosen from : SEQ ID NOs: 2, 4, 6, 8, 10, 12, 14, 16, 18, 20 or fragments or analogs thereof;
(b) a polypeptide having at least 95% identity to a second polypeptide comprising a sequence chosen from: SEQ ID NOs: 2, 4, 6, 8, 10, 12, 14, 16, 18, 20 or fragments or analogs thereof;
(c) a polypeptide comprising a sequence chosen from SEQ ID NOs: 2, 4, 6, 8, 10, 12, 14, 16, 18, 20 or fragments or analogs thereof;
(d) a polypeptide capable of generating antibodies having binding specificity for a polypeptide comprising a sequence chosen from SEQ ID NOs: 2, 4, 6, 8, 10, 12, 14, 16, 18, 20 or fragments or analogs thereof;
(e) an epitope bearing portion of a polypeptide comprising a sequence chosen from SEQ ID NOs: 2, 4, 6, 8, 10, 12, 14, 16, 18, 20 or fragments or analogs thereof;
(f) the polypeptide of (a), (b), (c), (d), (e) or (f) wherein the N-terminal Met residue is deleted;
(g) the polypeptide of (a), (b), (c), (d), (e) or (f) wherein the secretory amino acid sequence is deleted.

The present disclosure provides an isolated polypeptide comprising a polypeptide chosen from:
(a) a polypeptide having at least 70% identity to a second polypeptide comprising an amino acid sequence chosen from: SEQ ID NOs: 2, 4, 6, 8, 10, 12, 14, 16, 18, 20;
(b) a polypeptide having at least 95% identity to a second polypeptide comprising an amino acid sequence chosen from: SEQ ID NOs: 2, 4, 6, 8, 10, 12, 14, 16, 18, 20;
(c) a polypeptide comprising a sequence chosen from SEQ ID NOs: 2, 4, 6, 8, 10, 12, 14, 16, 18, 20;
(d) a polypeptide capable of generating antibodies having binding specificity for a polypeptide comprising a sequence chosen from SEQ ID NOs: 2, 4, 6, 8, 10, 12, 14, 16, 18, 20;
(e) an epitope bearing portion of a polypeptide comprising a sequence chosen from SEQ ID NOs : 2, 4, 6, 8, 10, 12, 14, 16, 18, 20;
(f) the polypeptide of (a), (b), (c), (d) or (e) wherein the N-terminal Met residue is deleted;
(g) the polypeptide of (a), (b), (c), (d), (e) or (f) wherein the secretory amino acid sequence is deleted.

According to one aspect, the present disclosure relates to polypeptides which comprise an amino acid sequence chosen from SEQ ID Nos : 2, 4, 6, 8, 10, 12, 14, 16, 18, 20 or fragments or analogs thereof.

According to one aspect, the present disclosure relates to polypeptides which comprise an amino acid sequence chosen from SEQ ID NO: 2, 4, 6, 8, 10, 12, 14, 16, 18 or 20.

Those skilled in the art will appreciate that the invention includes DNA molecules, i.e. polynucleotides and their complementary sequences that encode analogs such as mutants, variants, homologues and derivatives of such polypeptides, as described herein in the present patent application. The invention also includes RNA molecules corresponding to the DNA molecules of the invention. In addition to the DNA and RNA molecules, the disclosure includes the corresponding polypeptides and monospecific antibodies that specifically bind to such polypeptides.

In a further embodiment, the polypeptides in accordance with the present invention are antigenic.

In a further embodiment, the polypeptides in accordance with the present invention are immunogenic.

In a further embodiment, the polypeptides in accordance with the present invention can elicit an immune response in a host.

In a further embodiment, the present invention also relates to polypeptides which are able to raise antibodies having binding specificity to the polypeptides of the present invention as defined above.

An antibody that "has binding specificity" is an antibody that recognizes and binds the selected polypeptide but which does not substantially recognize and bind other molecules in a sample, e.g., a biological sample. Specific binding can be measured using an ELISA assay in which the selected polypeptide is used as an antigen.

In accordance with the present invention, "protection" in the biological studies is defined by a significant increase in the survival curve, rate or period. Statistical analysis using the Log rank test to compare survival curves, and Fisher exact test to compare survival rates and numbers of days to death, respectively, might be useful to calculate P values and determine whether the difference between the two groups is statistically significant. P values of 0.05 are regarded as not significant.

In an additional aspect of the disclosure there are provided antigenic/immunogenic fragments of the polypeptides of the invention, or of analogs thereof.

The fragments of the present disclosure should include one or more such epitopic regions or be sufficiently similar to such regions to retain their antigenic/immunogenic properties. Thus, for fragments according to the present disclosure the degree of identity is perhaps irrelevant, since they may be 100% identical to a particular part of a polypeptide or analog thereof as described herein. The present disclosure further provides fragments having at least 10 contiguous amino acid residues from the polypeptide sequences of the present invention. In one embodiment, at least 15 contiguous amino acid residues. In one embodiment, at least 20 contiguous amino acid residues.

The terms "fragment" or "variant," when referring to a polypeptide of the invention, mean a polypeptide which retains substantially at least one of the biological functions or activities of the polypeptide. Such a biological function or activity can be, *e.g*., any of those described above, and includes having the ability to react with an antibody, *i.e*., having a epitope-bearing peptide. Fragments or variants of the polypeptides, *e.g*. of SEQ ID NOS: 2, 4, 6, 8, 10, 12, 14, 16, 18 or 20, have sufficient similarity to those polypeptides so that at least one activity of the native polypeptides is retained. Fragments or variants of smaller polypeptides, *e.g*., of the polypeptides of SEQ ID NOS: 2, 4, 6, 8, 10, 12, 14, 16, 18 or 20, retain at least one activity (*e.g*., an activity expressed by a functional domain thereof, or the ability to react with an antibody or antigen-binding fragment of the invention) of a comparable sequence found in the native polypeptide.

The key issue, once again, is that the fragment retains the antigenic/immunogenic properties.

The skilled person will appreciate that analogs of the polypeptides of the invention will also find use in the context of the present invention, i.e. as antigenic/immunogenic material. Thus, for instance proteins or polypeptides which include one or more additions, deletions, substitutions or the like are encompassed by the present invention, in so far as this is encompassed by the appendant claims.

As used herein, "fragments", "analogs", "variants" or "derivatives" of the polypeptides of the invention include those polypeptides in which one or more of the amino acid residues are substituted with a conserved or non-conserved amino acid residue (preferably conserved) and which may be natural or unnatural and which are encompassed by the appendant claims. In one instance of the disclosure derivatives and analogs of polypeptides of the invention will have about 70% identity with those sequences illustrated in the figures or fragments thereof. That is, 70% of the residues are the same. In a further instance polypeptides will have greater than 80% identity. In a further embodiment, polypeptides will have greater than 85% identity. In a further embodiment, polypeptides will have greater than 90% identity. In a further embodiment, polypeptides will have greater than 95% identity. In a further embodiment, polypeptides will have greater than 99% identity. In a further embodiment, analogs of polypeptides of the invention will have fewer than about 20 amino acid residue substitutions, modifications or deletions and more preferably less than 10.

A variant of a polypeptide of the invention may be, *e.g.,* (i) one in which one or more of the amino acid residues are substituted with a conserved or non-conserved amino acid residue (preferably a conserved amino acid residue) and such substituted amino acid residue may or may not be one encoded by the genetic code, or (ii) one in which one or more of the amino acid residues includes a substituent group, or (iii) one in which the polypeptide is fused with another compound, such as a compound to increase the half-life of the polypeptide (for example, polyethylene glycol), or (iv) one in which additional amino acids are fused to the polypeptide, such as a leader or secretory sequence or a sequence which is employed for purification of the polypeptide, commonly for the purpose of creating a genetically engineered form of the protein that is susceptible to secretion from a cell, such as a transformed cell. The additional amino acids may be from a heterologous source, or may be endogenous to the natural gene.

These substitutions are those having a minimal influence on the secondary structure and hydropathic nature of the polypeptide. Preferred substitutions are those known in the art as conserved, i.e. the substituted residues share physical or chemical properties such as hydrophobicity, size, charge or functional groups. These include substitutions such as those described by Dayhoff, M. in Atlas of Protein Sequence and Structure 5, 1978 and by Argos, P. in EMBO J. 8, 779-785, 1989. For example, amino acids, either natural or unnatural, belonging to one of the following groups represent conservative changes:
ala, pro, gly, gln, asn, ser, thr, val;
cys, ser, tyr, thr;
val, ile, leu, met, ala, phe;
lys, arg, orn, his;
and phe, tyr, trp, his.

The preferred substitutions also include substitutions of D-enantiomers for the corresponding L-amino acids.

Variant polypeptides belonging to type (i) above include, *e.g*., muteins, analogs and derivatives. A variant polypeptide can differ in amino acid sequence by, *e.g*., one or more additions, substitutions, deletions, insertions, inversions, fusions, and truncations or a combination of any of these. Variant polypeptides belonging to type (ii) above include, *e.g*., modified polypeptides. Known polypeptide modifications include, but are not limited to, glycosylation, acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphatidylinositol, crosslinking, cyclization, disulfide bond formation, demethylation, formation of covalent crosslinks, formation of cystine, formation of pyroglutamate, formylation, gamma carboxylation, glycosylation, GPI anchor formatin, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination.

Such modifications are well-known to those of skill in the art and have been described in great detail in the scientific literature. Several particularly common modifications, glycosylation, lipid attachment, sulfation, gamma-carboxylation of glutamic acid residues, hydroxylation and ADP-ribosylation, for instance, are described in many basic texts, such as Proteins--Structure and Molecular Properties, 2nd ed., T.E. Creighton, W.H. Freeman and Company, New York (1993). Many detailed reviews are available on this subject, such as by Wold, F., Posttranslationail Covalent Modification of Proteins, B.C. Johnson, Ed., Academic Press, New York 1-12 (1983); Seifter et al. (1990) Meth. Enzymol. 182:626-646 and Rattan et al. (1992) Ann. N.Y. Acad. Sci. 663:48-62.

Variant polypeptides belonging to type (iii) are well-known in the art and include, *e.g*., PEGulation or other chemical modifications. Variants polypeptides belonging to type (iv) above include, *e.g*., preproteins or proproteins which can be activated by cleavage of the proprotein portion to produce an active mature polypeptide. Variants include a variety of hybrid, chimeric or fusion polypeptides. Typical example of such variants are discussed elsewhere herein.

Many other types of variants are known to those of skill in the art. For example, as is well known, polypeptides are not always entirely linear. For instance, polypeptides may be branched as a result of ubiquitination, and they may be circular, with or without branching, generally as a result of post-translation events, including natural processing events and events brought about by human manipulation which do not occur naturally. Circular, branched and branched circular polypeptides may be synthesized by non-translational natural processes and by synthetic methods.

Modifications or variations can occur anywhere in a polypeptide, including the peptide backbone, the amino acid side-chains and the amino or carboxyl termini. The same type of modification may be present in the same or varying degree at several sites in a given polypeptide. Also, a given polypeptide may contain more than one type of modification. Blockage of the amino or carboxyl group in a polypeptide, or both, by a covalent modification, is common in naturally-occurring and synthetic polypeptides. For instance, the aminoterminal residue of polypeptides made in *E*. *coli,* prior to proteolytic processing, is often N-formylmethionine. The modifications can be a function of how the protein is made. For recombinant polypeptides, for example, the modifications are determined by the host cell posttranslational modification capacity and the modification signals in the polypeptide amino acid sequence. Accordingly, when glycosylation is desired, a polypeptide can be expressed in a glycosylating host, generally a eukaryotic cell. Insect cells often carry out the same posttranslational glycosylations as mammalian cells and, for this reason, insect cell expression systems have been developed to efficiently express mammalian proteins having native patterns of glycosylation. Similar considerations apply to other modifications.

variant polypeptides can be fully functional or can lack function in one or more activities, *e.g.*, in any of the functions or activities described above. Among the many types of useful variations are, *e.g.*, those which exhibit alteration of catalytic activity. For example, one embodiment involves a variation at the binding site that results in binding but not hydrolysis, or slower hydrolysis, of cAMP. A further useful variation at the same site can result in altered affinity for cAMP. Useful variations also include changes that provide for affinity for another cyclic nucleotide. Another useful variation includes one that prevents activation by protein kinase A. Another useful variation provides a fusion protein in which one or more domains or subregions are operationally fused to one or more domains or subregions from another phosphodiesterase isoform or family.

In an alternative approach, the analogs could be fusion polypeptides, incorporating moieties which render purification easier, for example by effectively tagging the desired polypeptide. It may be necessary to remove the "tag" or it may be the case that the fusion polypeptide itself retains sufficient antigenicity to be useful.

The present invention provides a fusion polypeptide comprising the isolated polypeptide of any one of claims 11-15.

The percentage of homology is defined as the sum of the percentage of identity plus the percentage of similarity or conservation of amino acid type.

In one instance, analogs of polypeptides of the disclosure will have about 70% homology with those sequences illustrated in the figures or fragments thereof. In one instance, polypeptides will have greater than 80% homology. In a further embodiment, polypeptides will have greater than 85% homology. In a further embodiment, polypeptides will have greater than 90% homology. In a further embodiment, polypeptides will have greater than 95% homology. In a further embodiment, polypeptides will have greater than 99% homology. In a further embodiment, analogs of polypeptides of the invention will have fewer than about 20 amino acid residue substitutions, modifications or deletions and more preferably less than 10.

One can use a program such as the CLUSTAL program to compare amino acid sequences. This program compares amino acid sequences and finds the optimal alignment by inserting spaces in either sequence as appropriate. It is possible to calculate amino acid identity or homology for an optimal alignment. A program like BLASTx will align the longest stretch of similar sequences and assign a value to the fit. It is thus possible to obtain a comparison where several regions of similarity are found, each having a different score. Both types of identity analysis are contemplated in the present invention.

In an alternative approach, the analogs or derivatives could be fusion polypeptides, incorporating moieties which render purification easier, for example by effectively tagging the desired protein or polypeptide, it may be necessary to remove the "tag" or it may be the case that the fusion polypeptide itself retains sufficient antigenicity to be useful.

In an additional aspect of the disclosure there are provided antigenic/immunogenic fragments of the proteins or polypeptides or of analogs or derivatives thereof.

Thus, what is important for analogs, derivatives and fragments is that they possess at least a degree of the antigenicity/immunogenic of the protein or polypeptide from which they are derived.

Also included are polypeptides which have fused thereto other compounds which alter the polypeptides biological or pharmacological properties i.e. polyethylene glycol (PEG) to increase half-life; leader or secretory amino acid sequences for ease of purification; prepro- and pro- sequences; and (poly)saccharides.

Furthermore, in those situations where amino acid regions are found to be polymorphic, it may be desirable to vary one or more particular amino acids to more effectively mimic the different epitopes of the different Streptococcus strains.

Moreover, the polypeptides of the present invention can be modified by terminal -NH₂ acylation (eg. by acetylation, or thioglycolic acid amidation, terminal carboxy amidation, e.g. with ammonia or methylamine) to provide stability, increased hydrophobicity for linking or binding to a support or other molecule.

Also contemplated are hetero and homo polypeptide multimers of the polypeptide fragments and analogs. These polymeric forms include, for example, one or more polypeptides that have been cross-linked with cross-linkers such as avidin/biotin, gluteraldehyde or dimethylsuperimidate. Such polymeric forms also include polypeptides containing two or more tandem or inverted contiguous sequences, produced from multicistronic mRNAs generated by recombinant DNA technology. In a further embodiment, the present invention also relates to chimeric polypeptides which comprise one or more polypeptides or fragments or analogs thereof as defined in the figures of the present application.

The present invention provides a chimeric polypeptide comprising two or more polypeptides wherein each of the two or more polypeptides consists of an amino acid sequence chosen from SEQ ID NOS:4, 6, 8, and 12, provided that the two or more polypeptides are linked as to form a chimeric polypeptide.

In a further embodiment, the present disclosure also relates to chimeric polypeptides comprising two or more polypeptides having a sequence chosen from SEQ ID NOs : 2, 4, 6, 8, 10, 12, 14, 16, 18, 20 or fragments or analogs thereof; provided that the polypeptides are linked as to formed a chimeric polypeptide.

In a further embodiment, the present disclosure also relates to chimeric polypeptides comprising two or more polypeptides having a sequence chosen from SEQ ID NOs: 2, 4, 6, 8, 10, 12, 14, 16, 18 or 20 provided that the polypeptides are linked as to formed a chimeric polypeptide.

Preferably, a fragment, analog or derivative of a polypeptide of the invention will comprise at least one antigenic region i.e. at least one epitope.

In order to achieve the formation of antigenic polymers (i.e. synthetic multimers), polypeptides may be utilized having bishaloacetyl groups, nitroarylhalides, or the like, where the reagents being specific for thio groups. Therefore, the link between two mercapto groups of the different polypeptides may be a single bond or may be composed of a linking group of at least two, typically at least four, and not more than 16, but usually not more than about 14 carbon atoms.

In a particular instance, polypeptide fragments and analogs do not contain a methionine (Met) starting residue. Preferably, polypeptides will not incorporate a leader or secretory sequence (signal sequence). The signal portion of a polypeptide of the invention may be determined according to established molecular biological techniques. In general, the polypeptide of interest may be isolated from a Streptococcus culture and subsequently sequenced to determine the initial residue of the mature protein and therefore the sequence of the mature polypeptide.

In another instance, the polypeptides of the disclosure may be lacking an N-terminal leader peptide, and/or a transmembrane domain and/or a C-terminal anchor domain.

The present disclosure further provides a fragment of the polypeptide comprising substantially all of the extra cellular domain of a polypeptide which has at least 70% identify, preferably 80% identity, more preferably 95% identity, to a second polypeptide comprising a sequence chosen from SEQ ID NOs: 2, 4, 6, 8, 10, 12, 14, 16, 18 or 20 or fragments or analogs thereof, over the entire length of said sequence.

It is understood that polypeptides can be produced and/or used without their start codon (methionine or valine) and/or without their leader peptide to favor production and purification of recombinant polypeptides. It is known that cloning genes without sequences encoding leader peptides will restrict the polypeptides to the cytoplasm of E. coli and will facilitate their recovery (Glick, B.R. and Pasternak, J.J. (1998) Manipulation of gene expression in prokaryotes. In "Molecular biotechnology: Principles and applications of recombinant DNA", 2nd edition, ASM Press, Washington DC, p.109-143).

The present invention provides a vaccine comprising the polypeptide of any one of claims 11 to 15 and a pharmaceutically acceptable carrier, diluent or adjuvant. The invention further provides a vaccine comprising the fusion polypeptide according to claim 16 or the chimeric polypeptide of claim 17 and a pharmaceutically acceptable carrier, diluent or adjuvant.

According to another aspect of the disclosure, there are also provided (i) a composition of matter containing a polypeptide , together with a carrier, diluent or adjuvant; (ii) a pharmaceutical composition comprising a polypeptide and a carrier, diluent or adjuvant; (iii) a vaccine comprising a polypeptide and a carrier, diluent or adjuvant; (iv) a method for inducing an immune response against Streptococcus, in a host, by administering to the host, an immunogenically effective amount of a polypeptide to elicit an immune response, e.g., a protective immune response to Streptococcus; and particularly, (v) a method for preventing and/or treating a Streptococcus infection, by administering a prophylactic or therapeutic amount of a polypeptide to a host in need.

Before immunization, the polypeptides of the invention can also be coupled or conjugated to carrier proteins such as tetanus toxin, diphtheria toxin, hepatitis B virus surface antigen, poliomyelitis virus VP1 antigen or any other viral or bacterial toxin or antigen or any suitable proteins to stimulate the development of a stronger immune response. This coupling or conjugation can be done chemically or genetically. A more detailed description of peptide-carrier conjugation is available in Van Regenmortel, M.H.V., Briand J.P., Muller S., Plaué S., «Synthetic Polypeptides as antigens» in Laboratory Techniques in Biochemistry and Molecular Biology, Vol.19 (ed.) Burdou, R.H. & Van Knippenberg P.H. (1988), Elsevier New York.

According to another aspect of the disclosure, there are provided pharmaceutical compositions comprising one or more Streptococcus polypeptides of the invention in a mixture with a pharmaceutically acceptable adjuvant. Suitable adjuvants include (1) oil-in-water emulsion formulations such as MF59™, SAF™, Ribi™; (2) Freund's complete or incomplete adjuvant; (3) salts i.e. AlK(SO₄)₂, AlNa(SO₄)₂, AlNH₄(SO₄)₂, Al(OH)₃, AlPO₄, silica, kaolin; (4) saponin derivatives such as Stimulon™ or particles generated therefrom such as ISCOMs (immunostimulating complexes); (5) cytokines such as interleukins, interferons, macrophage colony stimulating factor (M-CSF), tumor necrosis factor (TNF) ; (6) other substances such as carbon polynucleotides i.e. poly IC and poly AU, detoxified cholera toxin (CTB)and E.coli heat labile toxin for induction of mucosal immunity; (7) liposomes. A more detailed description of adjuvants is available in a review by M.Z.I Khan et al. in Pharmaceutical Research, vol. 11, No. 1 (1994) pp2-11, and also in another review by Gupta et al., in Vaccine, Vol. 13, No. 14, pp1263-1276 (1995) and in WO 99/24578. Preferred adjuvants include QuilA™, QS21™, Alhydrogel™ and Adjuphos™.

Pharmaceutical compositions of the disclosure may be administered parenterally by injection, rapid infusion, nasopharyngeal absorption, dermoabsorption, or buccal or oral.

The term "pharmaceutical composition" is also meant to include antibodies. In accordance with the present disclosure, there is also provided the use of one or more antibodies having binding specificity for the polypeptides of the present invention for the treatment or prophylaxis of streptococcal infection and/or diseases and symptoms mediated by streptococcal infection.

Pharmaceutical compositions of the disclosure are used for the prophylaxis or treatment of streptococcal infection and/or diseases and symptoms mediated by streptococcal infection as described in Manual of Clinical Microbiology, P.R. Murray (Ed, in chief),E.J. Baron, M.A. Pfaller, F.C. Tenover and R.H. Yolken. ASM Press, Washington, D.C. seventh edition, 1999, 1773p.

In one aspect, the invention provides the polypeptide according to any one of Claims 11 to 15 for prophylactic or therapeutic treatment of Group B Streptococcus infection in a host susceptible to Group B Streptococcus infection. The invention further provides the vaccine according to any of Claims 18 to 19 for the prophylactic or therapeutic treatment of Group B Streptococcus infection in a host susceptible to Group B Streptococcus infection.

In one instance, pharmaceutical compositions are used for the prophylaxis or treatment of sepsis, meningitis, pneumonia, cellulitis, osteomyelitis, septic arthritis, endocarditis, epiglottis.

In one aspect, the invention provides the polypeptide according to any one of Claims 11 to 15 for prophylactic or therapeutic treatment of sepsis, meningitis, pneumonia, cellulitis, osteomyelitis, septic arthritis, endocarditis, or epiglottitis in a host. The invention further provides the vaccine according to any of Claims 18 to 19 for the prophylactic or therapeutic treatment of sepsis, meningitis, pneumonia, cellulitis, osteomyelitis, septic arthritis, endocarditis, or epiglottitis in a host. In one instance pharmaceutical compositions are used for the prophylaxis or treatment of mild urinary tract infection to life-threatening sepsis and meningitis, including also osteomyelitis, endocarditis, amniotis, endometritis, wound infections (postcesarean and postepisiotomy), cellulitis, fasciitis.

In one instance, pharmaceutical compositions are used for the prophylaxis or treatment of primary bacteremia but also skin of soft tissue infection, pneumonia, urosepsis, endocarditis, peritonitis, meningitis, empyema. Skin of soft tissue infections include cellulitis, infected peripheral ulcers, osteomyelitis, septic arthritis and decubiti or wound infections.

In one instance, pharmaceutical compositions are used for the treatment or prophylaxis of Streptococcus infection and/or diseases and symptoms mediated by Streptococcus infection, in particular group B Streptococcus (GBS or S.agalactiae), group A Streptococcus (Streptococcus pyogenes), S.pneumoniae, S.dysgalactiae, S.uberis, S.nocardia as well as Staphylococcus aureus. In a further embodiment, the Streptococcus infection is group B Streptococcus (GBS or S.agalactiae).

In a further instance, the disclosure provides a method for prophylaxis or treatment of Streptococcus infection in a host susceptible to Streptococcus infection comprising administering to said host a therapeutic or prophylactic amount of a composition of the invention.

In a further instance, the disclosure provides a method for prophylaxis or treatment of GBS infection in a host susceptible to GBS infection comprising administering to said host a therapeutic or prophylactic amount of a composition of the invention.

In a particular instance, pharmaceutical compositions are administered to those hosts at risk of Streptococcus infection such as infants, elderly and immunocompromised hosts.

As used in the present application, the term "host" includes animals. In a further embodiment, the animals are mammals. In a further embodiment, the animals are dairy herds. In a further embodiment, the mammal is human. In a further embodiment, the host is a pregnant woman. In a further embodiment, the host is a non-pregnant woman. In a further embodiment, the host is a neonate or an infant.

Pharmaceutical compositions are preferably in unit dosage form of about 0.001 to 100 µg/kg (antigen/body weight) and more preferably 0.01 to 10 µg/kg and most preferably 0.1 to 1 µg/kg 1 to 3 times with an interval of about 1 to 6 week intervals between immunizations.

Pharmaceutical compositions are preferably in unit dosage form of about 0.1 µg to 10 mg and more preferably µg to 1 mg and most preferably 10 to 100 µg 1 to 3 times with an interval of about 1 to 6 week intervals between immunizations.

According to another aspect of the disclosure, there are provided polynucleotides encoding polypeptides characterized by the amino acid sequence comprising SEQ ID NO: 2, 4, 6, 8, 10, 12, 14, 16, 18, 20 or fragments or analogs thereof.

In one embodiment, polynucleotides are those illustrated in SEQ ID No: 1, 3, 5, 7, 9, 11, 13, 15, 17, 19 which may include the open reading frames (ORF), encoding the polypeptides of the invention, in so far as this is encompassed by the appendant claims.

It will be appreciated that the polynucleotide sequences illustrated in the figures may be altered with degenerate codons yet still encode the polypeptides of the invention. Accordingly the present invention further provides polynucleotides which hybridize to the polynucleotide sequences herein above described (or the complement sequences thereof) having at least 85% identity between sequences. In one embodiment, at least 90% identity between sequences. In one embodiment, at least 95% identity. In a further embodiment, more than 97% identity.

In a further embodiment, polynucleotides are hybridizable under stringent conditions.

Suitable stringent conditions for hybridization can be readily determined by one of skilled in the art (see for example Sambrook et al., (1989) Molecular cloning : A Laboratory Manual, 2nd ed, Cold Spring Harbor, N.Y.; Current Protocols in Molecular Biology, (1999) Edited by Ausubel F.M. et al., John Wiley & Sons, Inc., N.Y.).

"Suitable stringent conditions", as used herein, means, for example, incubating a blot overnight (*e.g.*, at least 12 hours) with a long polynucleotide probe in a hybridization solution containing, e.g., about 5X SSC, 0.5% SDS, 100 µg/ml denatured salmon sperm DNA and 50% formamide, at 42°C. Blots can be washed at high stringency conditions that allow, e.g., for less than 5% bp mismatch (e.g., wash twice in 0.1X SSC and 0.1% SDS for 30 min at 65°C), thereby selecting sequences having, e.g., 95% or greater sequence identity.

Other non-limiting examples of suitable stringent conditions include a final wash at 65°C in aqueous buffer containing 30 mM NaCl and 0.5% SDS. Another example of suitable stringent conditions is hybridization in 7% SDS, 0.5 M NaPO₄, pH 7, 1 mM EDTA at 50°C, e.g., overnight, followed by one or more washes with a 1% SDS solution at 42°C. Whereas high stringency washes can allow for less than 5% mismatch, reduced or low stringency conditions can permit up to 20% nucleotide mismatch. Hybridization at low stringency can be accomplished as above, but using lower formamide conditions, lower temperatures and/or lower salt concentrations, as well as longer periods of incubation time.

In a further embodiment, the present disclosure provides polynucleotides that hybridize under stringent conditions to either
(a) a DNA sequence encoding a polypeptide or
(b) the complement of a DNA sequence encoding a polypeptide;
wherein said polypeptide comprises SEQ ID NO: 2, 4, 6, 8, 10, 12, 14, 16, 18, 20 or fragments or analogs thereof.

In a further instance, the present disclosure provides polynucleotides that hybridize under stringent conditions to either
(a) a DNA sequence encoding a polypeptide or
(b) the complement of a DNA sequence encoding a polypeptide;
wherein said polypeptide comprises SEQ ID NO: 2, 4, 6, 8, 10, 12, 14, 16, 18 or 20.

Also disclosed herein are: polynucleotides that hybridize under stringent conditions to either
(a) a DNA sequence encoding a polypeptide or
(b) the complement of a DNA sequence encoding a polypeptide;
wherein said polypeptide comprises at least 10 contiguous amino acid residues from a polypeptide comprising SEQ ID NO: 2, 4, 6, 8, 10, 12, 14, 16, 18, 20 or fragments or analogs thereof;

polynucleotides that hybridize under stringent conditions to either
(a) a DNA sequence encoding a polypeptide or
(b) the complement of a DNA sequence encoding a polypeptide;
wherein said polypeptide comprises at least 10 contiguous amino acid residues from a polypeptide comprising SEQ ID NO: 2, 4, 6, 8, 10, 12, 14, 16, 18 or 20.

In a further instance, polynucleotides are those encoding polypeptides of the invention illustrated in SEQ ID NO: 2, 4, 6, 8, 10, 12, 14, 16, 18, 20 or fragments or analogs thereof.

In a further instance, polynucleotides are those illustrated in SEQ ID NO: 1, 3, 5, 7, 9, 11, 13, 15, 17, 19 encoding polypeptides of the invention or fragments or analogs thereof.

In a further instance, polynucleotides are those encoding polypeptides of the invention illustrated in SEQ ID NO: 2, 4, 6, 8, 10, 12, 14, 16, 18, 20.

In a further instance, polynucleotides are those illustrated in SEQ ID NO: 1, 3, 5, 7, 9, 11, 13, 15, 17, 19 encoding polypeptides of the invention.

As will be readily appreciated by one skilled in the art, polynucleotides include both DNA and RNA.

The present invention also includes polynucleotides complementary to the polynucleotides described in the present application.

In a further instance, polynucleotides encoding polypeptides of the invention, or fragments, analogs or derivatives thereof, may be used in a DNA immunization method. That is, they can be incorporated into a vector which is replicable and expressible upon injection thereby producing the antigenic polypeptide in vivo. For example polynucleotides may be incorporated into a plasmid vector under the control of the CMV promoter which is functional in eukaryotic cells. Preferably the vector is injected intramuscularly.

According to another aspect, there is provided a process for producing polypeptides of the invention by recombinant techniques by expressing a polynucleotide encoding said polypeptide in a host cell and recovering the expressed polypeptide product. Alternatively, the polypeptides can be produced according to established synthetic chemical techniques i.e. solution phase or solid phase synthesis of oligopeptides which are ligated to produce the full polypeptide (block ligation).

General methods for obtention and evaluation of polynucleotides and polypeptides are described in the following references: Sambrook et al, Molecular Cloning: A Laboratory Manual, 2nd ed, Cold Spring Harbor, N.Y., 1989; Current Protocols in Molecular Biology, Edited by Ausubel F.M. et al., John Wiley and Sons, Inc. New York; PCR Cloning Protocols, from Molecular Cloning to Genetic Engineering, Edited by White B.A., Humana Press, Totowa, New Jersey, 1997, 490 pages; Protein Purification, Principles and Practices, Scopes R.K., Springer-Verlag, New York, 3rd Edition, 1993, 380 pages; Current Protocols in Immunology, Edited by Coligan J.E. et al., John Wiley & Sons Inc., New York.

For recombinant production, host cells are transfected with vectors which encode the polypeptides of the invention, and then cultured in a nutrient media modified as appropriate for activating promoters, selecting transformants or amplifying the genes. Suitable vectors are those that are viable and replicable in the chosen host and include chromosomal, non-chromosomal and synthetic DNA sequences e.g. bacterial plasmids, phage DNA, baculovirus, yeast plasmids, vectors derived from combinations of plasmids and phage DNA. The polypeptide sequence may be incorporated in the vector at the appropriate site using restriction enzymes such that it is operably linked to an expression control region comprising a promoter, ribosome binding site (consensus region or Shine-Dalgarno sequence), and optionally an operator (control element). One can select individual components of the expression control region that are appropriate for a given host and vector according to established molecular biology principles (Sambrook et al, Molecular Cloning: A Laboratory Manual, 2nd ed, Cold Spring Harbor, N.Y., 1989; Current Protocols in Molecular Biology, Edited by Ausubel F.M. et al., John Wiley and Sons, Inc. New York). Suitable promoters include but are not limited to LTR or SV40 promoter, E.coli lac, tac or trp promoters and the phage lambda P_{L} promoter. Vectors will preferably incorporate an origin of replication as well as selection markers i.e. ampicilin resistance gene. Suitable bacterial vectors include pET, pQE70, pQE60, pQE-9, pD10 phagescript, psix174, pbluescript SK, pbsks, pNH8A, pNH16a, pNH18A, pNH46A, ptrc99a, pKK223-3, pKK233-3, pDR540, pRIT5 and eukaryotic vectors pBlueBacIII, pWLNEO, pSV2CAT, pOG44, pXT1, pSG, pSVK3, pBPV, pMSG and pSVL. Host cells may be bacterial i.e. E.coli, Bacillus subtilis, Streptomyces; fungal i.e. Aspergillus niger, Aspergillus nidulins; yeast i.e. Saccharomyces or eukaryotic i.e. CHO, COS.

Upon expression of the polypeptide in culture, cells are typically harvested by centrifugation then disrupted by physical or chemical means (if the expressed polypeptide is not secreted into the media) and the resulting crude extract retained to isolate the polypeptide of interest. Purification of the polypeptide from culture media or lysate may be achieved by established techniques depending on the properties of the polypeptide i.e. using ammonium sulfate or ethanol precipitation, acid extraction, anion or cation exchange chromatography, phosphocellulose chromatography, hydrophobic interaction chromatography, hydroxylapatite chromatography and lectin chromatography. Final purification may be achieved using HPLC.

The polypeptides may be expressed with or without a leader or secretion sequence. In the former case the leader may be removed using post-translational processing (see US 4,431,739; US 4,425,437; and US 4,338,397) or be chemically removed subsequent to purifying the expressed polypeptide.

According to a further aspect, the polypeptides of the invention may be used in a diagnostic test for Streptococcus infection, in particular group B Streptococcus infection.

Several diagnostic methods are possible, for example detecting Streptococcus organism in a biological sample previously obtained from a host, the following procedure may be followed:
a) incubating an antibody or fragment thereof reactive with a Streptococcal polypeptide of the invention with the biological sample to form a mixture; and
b) detecting specifically bound antibody or bound fragment in the mixture which indicates the presence of Streptococcus.

Such diagnostic methods are encompassed by the invention, in so far as this is encompassed by the appendant claims.

Alternatively, a method for the detection of antibody specific to a Streptococcus antigen and in particular group B Streptococcus antigen in a biological sample previously obtained from a host containing or suspected of containing said antibody may be performed as follows:
a) incubating one or more Streptococcal polypeptides of the invention or fragments thereof with the biological sample to form a mixture; and
b) detecting specifically bound antigen or bound fragment in the mixture which indicates the presence of antibody specific to Streptococcus. Such diagnostic methods are encompassed by the invention, in so far as this is encompassed by the appendant claims.

One of skill in the art will recognize that this diagnostic test may take several forms, including an immunological test such as an enzyme-linked immunosorbent assay (ELISA), a radioimmunoassay or a latex agglutination assay, essentially to determine whether antibodies specific for the polypeptide are present in an organism.

The DNA sequences encoding polypeptides of the invention may also be used to design DNA probes for use in detecting the presence of Streptococcus in a biological sample suspected of containing such bacteria. The detection method of this invention comprises:
a) obtaining the biological sample from a host;
b) incubating one or more DNA probes having a DNA sequence encoding a polypeptide of the invention or fragments thereof with the biological sample to form a mixture; and
c) detecting specifically bound DNA probe in the mixture which indicates the presence of Streptococcus bacteria.

The DNA probes may also be used for detecting circulating Streptococcus i.e. Streptococcus nucleic acids in a sample, for example using a polymerase chain reaction, as a method of diagnosing Streptococcus infections. The probe may be synthesized using conventional techniques and may be immobilized on a solid phase, or may be labelled with a detectable label. A preferred DNA probe is an oligomer having a sequence complementary to at least about 6 contiguous nucleotides of the Streptococcus polypeptides of the invention.

Another diagnostic method for the detection of Streptococcus in a host comprises:
a) labelling an antibody reactive with a polypeptide of the invention or fragment thereof with a detectable label;
b) administering the labelled antibody or labelled fragment to the host; and
c) detecting specifically bound labelled antibody or labelled fragment in the host which indicates the presence of Streptococcus.

A further aspect of the disclosure is the use of the Streptococcus polypeptides of the invention as immunogens for the production of specific antibodies for the diagnosis and in particular the treatment of Streptococcus infection. Suitable antibodies may be determined using appropriate screening methods, for example by measuring the ability of a particular antibody to passively protect against Streptococcus infection in a test model. One example of an animal model is the mouse model described in the examples herein. The antibody may be a whole antibody or an antigen-binding fragment thereof and may belong to any immunoglobulin class. The antibody or fragment may be of animal origin, specifically of mammalian origin and more specifically of murine, rat or human origin. It may be a natural antibody or a fragment thereof, or if desired, a recombinant antibody or antibody fragment. The term recombinant antibody or antibody fragment means antibody or antibody fragment which was produced using molecular biology techniques. The antibody or antibody fragments may be polyclonal, or preferably monoclonal. It may be specific for a number of epitopes associated with the Streptococcus polypeptides but is preferably specific for one.

A further aspect disclosed herein is the use of the antibodies directed to the polypeptides of the invention for passive immunization. One could use the antibodies described in the present application. Suitable antibodies may be determined using appropriate screening methods, for example by measuring the ability of a particular antibody to passively protect against Streptococcus infection in a test model. One example of an animal model is the mouse model described in the examples herein. The antibody may be a whole antibody or an antigen-binding fragment thereof and may belong to any immunoglobulin class. The antibody or fragment may be of animal origin, specifically of mammalian origin and more specifically of murine, rat or human origin. It may be a natural antibody or a fragment thereof, or if desired, a recombinant antibody or antibody fragment. The term recombinant antibody or antibody fragment means antibody or antibody fragment which was produced using molecular biology techniques. The antibody or antibody fragments may be polyclonal, or preferably monoclonal. It may be specific for a number of epitopes associated with the Streptococcus polypeptides but is preferably specific for one.

The use of a polynucleotide disclosed herein in genetic immunization will preferably employ a suitable delivery method or system such as direct injection of plasmid DNA into muscles [Wolf et al. H M G (1992) 1: 363; Turnes et al., Vaccine (1999), 17 : 2089; Le et al., Vaccine (2000) 18 : 1893; Alves et al., Vaccine (2001) 19 : 788], injection of plasmid DNA with or without adjuvants [Ulmer et al., Vaccine (1999) 18: 18; MacLaughlin et al., J. Control Release (1998) 56: 259; Hartikka et al., Gene Ther. (2000) 7: 1171-82; Benvenisty and Reshef, PNAS USA (1986) 83:9551; Singh et al., PNAS USA (2000) 97: 811], targeting cells by delivery of DNA complexed with specific carriers [wa et al., J Biol Chem (1989) 264: 16985; Chaplin et al., Infect. Immun. (1999) 67: 6434], injection of plasmid complexed or encapsulated in various forms of liposomes [Ishii et al., AIDS Research and Human Retroviruses (1997) 13: 142; Perrie et al., Vaccine (2001) 19: 3301], administration of DNA with different methods of bombardment [Tang et al., Nature (1992) 356: 152; Eisenbraun et al., DNA Cell Biol (1993) 12: 791; Chen et al., Vaccine (2001) 19: 2908], and administration of DNA with lived vectors [Tubulekas et al., Gene (1997) 190: 191; Pushko et al., Virology (1997) 239: 389; Spreng et al. FEMS (2000) 27: 299; Dietrich et al., Vaccine (2001) 19: 2506].

In a further instance, the disclosure provides a method for prophylactic or therapeutic treatment of Streptococcus infection in a host susceptible to Streptococcus infection comprising administering to the host a prophylactic or therapeutic amount of a pharmaceutical composition of the invention.

In a further embodiment, the invention in so far as is encompassed by the appendant claims provides the use of a pharmaceutical method for the prophylactic or therapeutic treatment of streptococcal bacterial infection in a host susceptible to streptococcal infection comprising administering to said host a therapeutic or prophylactic amount of a composition of the invention.

In a further embodiment, the invention provides a kit comprising a polypeptide of the invention for detection or diagnosis of streptococcal infection.

Unless otherwise defined, all technical and scientific terms used herein have the same meaning as commonly understood by one of ordinary skill in the art to which this invention belongs. In case of conflict, the present specification, including definitions, will control.

It is understood that this invention relates to SEQ ID NOs: 3, 4, 5, 6, 7, 8, 11 and 12.

However, references to other sequence identifying numbers are retained in the following examples to assist with the understanding of the invention.

### Example 1

This example describes the cloning of truncated sip gene products by polymerase chain reaction (PCR) and the expression of truncated molecules.

Fragments of Group,B streptococcal sip (SEQ ID NO: 42 from PCT WO 99/42588) gene were amplified by PCR (DNA Thermal Cycler GeneAmp PCR system 2400 Perkin Elmer) from genomic DNA of serotype I a/c Group B streptococcal strain C388/90 using pairs of oligonucleotide primers that contained base extensions for the addition of restriction sites and methionine (Table 1). The methionine was added for C-terminal and internal truncated Sip polypeptide. PCR products were purified from agarose gel using a QIAquick gel extraction kit from QIAgen following the manufacturer's instructions, and digested with restriction endonucleases. The pET vector (Novagen, Madison, WI) was digested with the same endonucleases and purified from agarose gel using a QIAquick gel extraction kit from QIAgen. The digested PCR products were ligated to one of the following linearized pET expression plasmid, pET21 or pET32. The ligated product was transformed into E. coli strain DH5a [F⁻φ80d*lac*ZΔM15 Δ(*lac*ZYA-*arg*F) U169 endA1 *rec*A1 *hsd*R17 (rₖ⁻ mₖ⁺) *deo*R *thi*-1 *pho*A *supE44* λ⁻*gyr*A96 *rel*A1] (Gibco BRL, Gaithersburg, MD) according to the manufacturer's recommendations. Recombinant pET plasmids (rpET) containing sip gene fragments were purified using a QIAgen plasmid kit and their DNA insert was sequenced (Taq Dye Deoxy Terminator Cycle Sequencing kit, ABI, Foster City, CA). Each of the resultant plasmid constructs was used to transform by electroporation (Gene Pulser II apparatus, BIO-RAD Labs, Mississauga, Ontario, Canada) E. coli strain BL21(DE3) (F⁻ *omp*T *hsd*S*_{B}*(r⁻*_{B}*m⁻*_{B}*) *ga1 dcm* (DE3)) or AD494 (DE3) [*Δara-leu7697 ΔlacX74 ΔphoA PvuII phoR ΔmalF3* F' [*lac*⁺(*lacI^{q}*) *pro]* trxB: : Kan (DE3)] (Novagen). In these strains of E. coli, the T7 promotor controlling expression of the recombinant polypeptide is specifically recognized by the T7 RNA polymerase (present on the λDE3 prophage) whose gene is under the control of the lac promotor which is inducible by IPTG. The transformants were grown at 37°C with agitation at 250 rpm in LB broth (peptone 10g/L, yeast extract 5g/L, NaCl 10g/L) containing 100 µg of carbenicillin (Sigma-Aldrich Canada Ltd., Oakville, Ontario, Canada) per ml until the A₆₀₀ reached a value of 0.6. In order to induce the production of Group B streptococcal truncated Sip recombinant polypeptides, the cells were incubated for 3 additional hours in the presence of IPTG at a final concentration of 1 mM. Induced cells from a 500 ml culture were pelleted by centrifugation and frozen at -80°C. The expressed recombinant polypeptides were purified from supernatant fractions obtained from centrifugation of sonicated IPTG-induced E. coli cultures using a His-Bind metal chelation resin (QIAgen, Chatsworth, CA). The gene products generated are listed in the Table 2. The quantities of recombinant polypeptides obtained from the soluble fraction of E. coli was estimated by MicroBCA (Pierce, Rockford, Illinois).

**Table 1. List of PCR oligonucleotide primers**

| **Primer** | **Restriction sites** | **Sequence 5' - 3'** | **SEQ ID No** |
|---|---|---|---|
| DMAR41 | *Nco*I | catgccatggcagggctccaacctcatgtt | 21 |
| DMAR54 | *Nco*I | catgccatggcagctaatgaacaggtatcaacagc | 22 |
| DMAR55 | *Xho*I | gaaactcgagtgcattttcaggatgtgcagctac | 23 |
| DMAR207 | *Bgl*II | gcccagatctgggtaaaaaccaagcacttgg | 24 |
| DMAR208 | *Bgl*II | gcccagatctggttatctggcaacaaaagttttac | 25 |
| DMAR1451 | *Hind*III | cgggaagcttattatttgttaaatgatacgtgaaca | 26 |
| DMAR1452 | *Nco*I | caagccatgggtatgacaccagaagcagcaaca | 27 |
| DMAR1453 | *Xho*I | accgctcgagtttgttaaatgatacgtgaacatggtca | 28 |
| DMAR1454 | *Nco*I | ccatccatggtcagtcagtcaacaacagtatcaccag | 29 |
| DMAR1455 | *Nco*I | aatgccatggttcctgtgactacgacttcaacagc | 30 |
| DMAR1456 | *Xho*I | aactcgaggctgctaaacctttaccatgatcacct | 31 |
| DMAR1457 | *Nde*I | atgggaattccatatgaaaatgaataaaaaggtactat tgacatc | 32 |
| DMAR1458 | *Xho*I | atagctcgagtgacgtctgagttggtttaacttcc | 33 |

**Table 2. Lists of truncated sip gene products generated from GBS strain C388/90**

| **Polypeptide designation** | **PCR-primer sets** | **Identification (encoded amino acids)** | **Cloning vector** |
|---|---|---|---|
| Δsip-1 | DMAR1457-DMAR1458 | Sip N'end (1-214) | pET-21a(+) |
| ΔSip-2 | DMAR1453-DMAR1454 | Sip C'end (215-434) | pET-21d(+) |
| ΔSip-3 | DMAR1452-DMAR1453 | Sip C'end (146-434) | pET-21d(+) |
| ΔSip-4 | DMAR1453-DMAR1455 | Sip C'end (272-434) | pET-21d(+) |
| ΔSip-5 | DMAR1456-DMAR1457 | Sip N'end (1-360) | pET-21a(+) |
| ΔSip-6 | DMAR1453-DMAR54 | Sip N'end (184-434) | pET-21d(+) |
| ΔSip-7 | DMAR1452-DMAR55 | Sip internal (146-322) | pET-21d(+) |
| ΔSip-8 | DMAR1453-DMAR41 | Sip C'end (322-434) | pET-21d(+) |
| ΔSip-9 | DMAR207-DMAR1451 | Sip C'end (366-434) | pET-32a(+) |
| ΔSip-10 | DMAR208-DMAR1451 | Sip C'end (391-434) | pET-32a(+) |

### EXAMPLE 2

This example illustrates the reactivity of the His-tagged truncated Sip recombinant polypeptides with antibodies present in human sera.

As shown in Table 3, ΔSip-2 (215-434), ΔSip-3 (146-434), and ΔSip-4 (272-434) His-tagged recombinant polypeptides were best recognized in immunoblots by the antibodies present in the pool of human sera. This is an important result since it clearly indicates that humans which are normally in contact with GBS do develop antibodies that are specific to the C-terminal portion of the polypeptide (aa 215-434). These particular human antibodies might be implicated in the protection against GBS infection.

**Table 3. Reactivity in immunoblots of antibodies present in human sera with truncated Sip polypeptides.**

| **Purified recombinant polypeptide I.D.¹** | **Reactivity with human sera²** |
|---|---|
| Sip (1-434) | +++ |
| ΔSip-1 (1-214) | + |
| ΔSip-2 (215-434) | +++ |
| ΔSip-3 (146-434) | +++ |
| ΔSip-4 (272-434) | ++ |
| ΔSip-5 (1-360) | + |

| | |
|---|---|
| ¹His-tagged recombinant polypeptides produced and purified as described in Example 1 were used to perform the immunoblots. ²Sera collected from humans were pooled and diluted 1/500 to perform the immunoblots. | |

### EXAMPLE 3

This example illustrates the binding at the surface of intact GBS cells of antibodies directed against truncated Sip polypeptides.

Bacterial cells were grown to early exponential phase in Todd-Hewitt broth (THB: Difco Laboratories, Detroit, Mich.) and the OD₆₀₀ₙₘ was adjusted with THB to 0.15 (corresponding to ∼10⁸ CFU/ml). Ten µl of mouse truncated Sip-specific or control sera were added to 1 ml of the bacterial suspension. The tubes containing the bacterial and sera suspensions were incubated for 2 h at 4°C under gentle rotation. Samples were washed 3 times in blocking buffer [phosphate-buffered saline (PBS) containing 2% (wt/vol) bovine serum albumin (BSA: Sigma Chemical Co., St. Louis, Mo.)], and then 1 ml of goat fluorescein (FITC)-conjugated anti-mouse IgG + IgM (Jackson ImmunoResearch Laboratories, Mississauga, Ontario, Canada) diluted in blocking buffer was added. After a further incubation of 60 min at room temperature, samples were washed 3 times in blocking buffer and fixed with 0.3 % formaldehyde in PBS buffer for 18 h at 4°C. Cells were washed 2 times in PBS buffer and resuspended in 0.5 ml of PBS buffer. Cells were kept in the dark at 4°C until being analyzed by flow cytometry (Epics® XL; Beckman Coulter Inc., Fullerton, Calif.).

Flow cytometric analysis revealed that ΔSip-2 (215-434), ΔSip-3 (146-434), and ΔSip-4 (272-434)-specific antibodies efficiently recognized their corresponding surface-exposed epitopes on the homologous (C388/90) GBS strain tested (Table 4). It was determined that more than 90 % of the 10,000 GBS cells analyzed were labeled with the antibodies present in these sera. In addition, antibodies present in the pool of ΔSip-2 (215-434), ΔSip-3 (146-434), and ΔSip-4 (272-434)-specific sera attached at the surface of the serotype III GBS strain NCS 954 (Table 4). It was also determined that more than 80% of the 10,000 cells of this strain were labeled by the specific antibodies. These observations clearly demonstrate that the C-terminal portion of the Sip polypeptide is accessible at the surface, where it can be easily recognized by antibodies. Anti-GBS antibodies were shown to play an important role in the protection against GBS infection. Indeed, we have demonstrated that Sip-specific antibodies efficiently cross the transplacental barrier and thus confer protective immunity against GBS infections (Martin et al. Abstr. 101th Gen. Meet. Am. Soc. Microbiol. 2001).

**Table 4. Evaluation of the attachment of truncated Sip-specific antibodies at the surface of intact GBS cells.**

| **Serum identification¹** | **Strain C388** | **8/90 (I a/c)** | | **Strain NCS** | **954 (III)** |
|---|---|---|---|---|---|
| | % of labeled cells² | Fluorescence Index³ | | % of labeled cells | Fluorescence Index |
| Pool of ΔSip-1-specific sera | 5.1 | 1.2 | | 10.0 | 1.6 |
| Pool of ΔSip-2-specific sera | 95.6 | 18.7 | | 87.7 | 14.2 |
| Pool of ΔSip-3-specific sera | 96.0 | 19.4 | | 87.7 | 13.3 |
| Pool of ΔSip-4-specific sera | 94.2 | 17.2 | | 84.2 | 11.6 |
| Pool of ΔSip-5-specific sera | 21.6 | 2.2 | | 5.2 | 1.3 |
| Pool of positive control serum⁴ | 95.4 | 24.1 | | 85.4 | 12.3 |
| Pool of negative control sera⁵ | 1.0 | 1.0 | | 1.0 | 1.0 |

| | | | | | |
|---|---|---|---|---|---|
| ¹The mice were injected subcutaneously three times at three-week intervals with 20 µg of purified recombinant polypeptides mixed with 20 µg of QuilA adjuvant. The sera were diluted 1/100. ²% of labeled cells out of the 10,000 cells analyzed. ³The fluorescence index was calculated as the median fluorescence value obtained after labeling the cells with an immune serum divided by the fluorescence value obtained for a control mouse serum. A fluorescence value of 1 indicated that there was no binding of antibodies at the surface of intact GBS cells. ⁴Serum obtained from a mouse immunized with 20 µg of purified Sip polypeptide from GBS strain C388/90 was diluted 1/100 and used as a positive control for the assay. ⁵Sera collected from unimmunized or sham-immunized mice were pooled, diluted 1/100, and used as negative controls for this assay. | | | | | |

### EXAMPLE 4

This example illustrates the protection of mice against fatal Group B streptococcal infection induced by immunization with purified truncated Sip recombinant polypeptides.

Groups of 8 female CD-1 mice (Charles River) were immunized subcutaneously three times at three-week intervals with 20 µg of truncated Sip polypeptides that were produced and purified as described in Example 1 in presence of 20 µg of QuilA adjuvant (Cedarlane Laboratories Ltd, Hornby, Ontario, Canada). The control mice were injected with QuilA adjuvant alone in PBS. Blood samples were collected from the orbital sinus on day 1, 21, and 42 prior to each immunization and 14 days (day 56) following the third injection. One weeks later the mice were challenged with approximately 3x10⁵ CFU of the Group B streptococcal strain C388/90 (Ia/c). Samples of the Group B streptococcal challenge inoculum were plated on blood agar plates to determine the CFU and to verify the challenge dose. Deaths were recorded for a period of 7 days. More than 60% of the mice immunized with either ΔSip-2 (215-434), ΔSip-3 (146-434), ΔSip-4 (272-434), and ΔSip-6 (184-434) recombinant polypeptides were protected against a lethal challenge with GBS. On the contrary, immunization of mice with adjuvant only, ΔSip-1 (1-214), or ΔSip-5 (1-360) did not confer such protection (Table 5).The survival rate determined for the groups of mice immunized with ΔSip-2 (215-434), ΔSip-3 (146-434), ΔSip-4 (272-434), and ΔSip-6 (184-434) were shown to be statistically different from the control group by the Fisher's exact test.

**Table 5. Ability of recombinant truncated Sip polypeptides to elicit protection against GBS strain C388/90 (I a/c)**

| **Groups** | **No. mice surviving** | **% survival** |
|---|---|---|
| ΔSip-1 (1-214) | 0/5 | 0 |
| ΔSip-2 (215-434) | 3/5 | 60* |
| ΔSip-3 (146-434) | 3/5 | 60* |
| ΔSip-4 (272-434) | 3/4 | 75* |
| ΔSip-5 (1-360) | 2/5 | 40 |
| ΔSip-6 (184-434) | 7/8 | 88* |
| QuilA | 0/5 | 0 |

| | | |
|---|---|---|
| * Fisher's exact test.; p<0 . 05 | | |

### EXAMPLE 5

This example describes the isolation of monoclonal antibodies (Mabs) and the use of these Mabs to characterize the Sip polypeptide epitopes.

Female CD1 mice (Charles River) were immunized subcutaneously with Δsip-3 gene product from GBS strain C388/90 in presence of 20 µg of QuilA adjuvant (Cedarlane Laboratories Ltd, Hornby, Canada). A group of mice were immunized three times at three-week intervals with 20 µg of affinity purified ΔSip-3 polypeptide. Three to four days before fusion, mice were injected intravenously with 10 µg of the respective antigen suspended in PBS alone. Hybridomas were produced by fusion of spleen cells with non-secreting SP2/0 myeloma cells as previously described by Hamel et al. [J. Med. Microbiol., 23, pp163-170 (1987)]. Culture supernatants of hybridomas were initially screened by enzyme-linked-immunoassay according to the procedure described by Brodeur et al. (2000) using plates coated with preparations of purified recombinant polypeptides or suspensions of heat-killed GBS cells. Positive hybridomas selected on the basis of ELISA reactivity with a variety of antigens were then cloned by limiting dilutions, expanded and frozen.

Hybridomas were tested by ELISA and Western immunoblotting against sip gene products, and by cytofluorometry assay against GBS strain C388/90 (serotype Ia/c) in order to characterize the epitopes recognized by the Mabs. The results obtained from the immunoreactivity studies of the Mabs (Table 6 and Table 7) are in agreement with the surface accessibility obtained with truncated Sip polypeptides. Indeed, the most accessible Mabs recognized the C-terminal region (215-434) of the Sip polypeptide. Particularly, data revealed the presence of at least four distinct surface-exposed and potentially protective epitopes on the Sip polypeptide. These regions were determined to be located to amino acids 215-272, 272-322, 360-366, and 391-434. On the contrary, epitopes located at the N-terminal portion comprising amino acids 1 to 214 were internal and not accessible to antibodies.

**Table 6. Reactivity of Sip-immunoreactive Mabs with a panel of sip gene products.**

| Mabs | Sip | ΔSip-1 (1-214aa) | ΔSip-2 (215-434aa) | ΔSip-3 (146-434aa) | ΔSip-4 (272-434aa) | ΔSip-5 (1-360aa) | ΔSip-6 (184-434aa) | ΔSip-7 (146-322aa) | ΔSip-8 (322-434aa) | ΔSip-9 (366-434aa) | ΔSip-10 (391-434aa) |
|---|---|---|---|---|---|---|---|---|---|---|---|
| 1F7 | + | - | + | + | + | - | + | - | + | - | - |
| 3B7 | + | - | + | + | + | - | + | - | + | + | + |
| 4F2 | + | - | + | + | + | - | + | - | + | - | - |
| 5E5 | + | + | - | + | - | + | + | + | - | NT* | NT |
| 5F11 | + | - | + | + | + | - | + | - | + | - | - |
| 6F3 | + | - | + | + | + | + | + | + | - | NT | NT |
| 8E3 | + | - | + | + | + | + | + | + | - | NT | NT |
| 8F6 | + | + | - | + | - | + | - | + | - | NT | NT |
| 9C7 | + | - | + | + | + | - | + | - | + | - | - |
| 11C9 | + | - | + | + | + | + | + | + | - | NT | NT |
| 11D2 | + | - | + | + | + | - | + | - | + | - | - |
| 11E10 | + | - | + | + | + | - | + | - | + | - | - |
| 12G10 | + | - | + | + | - | + | + | + | - | NT | NT |
| 13D12 | + | - | + | + | + | - | + | - | + | + | + |
| 14A2 | + | - | + | + | + | - | + | - | + | + | + |
| 14H4 | + | - | + | + | + | - | + | - | + | + | + |
| 14H8 | + | - | + | + | + | - | + | - | + | - | - |
| 17C 10 | + | + | - | + | - | + | - | + | - | NT | NT |
| 18A8 | + | - | + | + | + | - | + | - | + | + | + |
| 18H10 | + | - | + | + | + | - | + | - | + | - | - |
| 20A2 | + | - | + | + | - | + | + | + | - | NT | NT |
| 20G5 | + | - | + | + | + | - | + | - | + | - | - |

| | | | | | | | | | | | |
|---|---|---|---|---|---|---|---|---|---|---|---|
| *NT: not tested. | | | | | | | | | | | |

**Table 7. Evaluation of Sip-immunoreactive Mabs attachment at the surface of intact GBS cells.**

| Mabs | **Recognized epitope (aa)¹** | % of labeled cells² | Fluorescence index³ |
|---|---|---|---|
| 17C10 | 146-184 | 0.7 | 1.6 |
| 8F6 | 146-184 | 1.1 | 1.8 |
| 5E5 | 184-215 | 5.7 | 1.9 |
| 12G10 | 215-272 | 42.1 | 7.1 |
| 20A2 | 215-272 | 1.1 | 1.8 |
| 6F3 | 272-322 | 25.6 | 4.9 |
| 8E3 | 272-322 | 28.6 | 5.2 |
| 11C9 | 272-322 | 39.7 | 5.8 |
| 1F7 | 360-366 | 78.9 | 7.5 |
| 4F2 | 360-366 | 97.9 | 7.2 |
| 5F11 | 360-366 | 43.1 | 2.2 |
| 9C7 | 360-366 | 93.8 | 6.1 |
| 11D2 | 360-366 | 87.1 | 11.9 |
| 11E10 | 360-366 | 45.8 | 2.3 |
| 14H8 | 360-366 | 90.8 | 4.8 |
| 18H10 | 360-366 | 98.0 | 8.4 |
| 20G5 | 360-366 | 96.5 | 6.9 |
| 3B7 | 391-434 | 98.3 | 10.4 |
| 13D12 | 391-434 | 90.0 | 10.4 |
| 14A2 | 391-434 | 98.4 | 10.8 |
| 14H4 | 391-434 | 97.5 | 10.0 |
| 18A8 | 391-434 | 97.7 | 11.7 |
| Negative control Mab⁴ | - | 1.5 | 1.0 |
| Pool of positive control serum⁵ | - | 98.7 | 25.0 |

| | | | |
|---|---|---|---|
| ¹Epitopes have been determined by the Mabs reactivity with truncated Sip polypeptides (see Table 6). ²% of labeled cells out of the 10,000 cells analyzed. ³The fluorescence index was calculated as the median fluorescence value obtained after labeling the cells with a Mab or immune serum divided by the fluorescence value obtained for a control Mab. A fluorescence value of 1 indicated that there was no binding of antibodies at the surface of intact GBS cells. ⁴Irrevalant Mab was not diluted and was used as negative controls for this assay. ⁵Serum obtained from a mouse immunized with 20 µg of purified Sip polypeptide from GBS strain C388/90 was diluted 1/100 and was used as a positive control for the assay. | | | |

### Example 6

This example illustrates the protection of mice against fatal Group B streptococcal infection induced by passive immunization with Sip-specific Mabs.

The protective potential of Sip-specific Mabs to protect neonates against infection was evaluated by passive administration of semi-purified Mabs antibodies. Pregnant mice on day 16 of gestation were injected intravenously (i.v.) with 500 µl of semi-purified Mabs antibodies or partially purified rabbit Sip-specific antibodies. Six control pregnant mice received the same volume of semi-purified irrelevant Mab. The pups were challenged subcutaneously (s.c.) between 24 h to 48 h after birth with a lethal dose of 3-4x10⁴ cfu from the serotype Ia/c GBS strain C388/90. The survival data are presented in Table 8. Administration to pregnant of a combination of two Sip-specific Mabs, 6F3 and 11D2, protected 65% (15/23) of the pups against a lethal GBS challenge. Comparable survival of the pups was not observed when the pregnant mice received one Sip-specific Mab.

**Table 8. Passive protection of neonatal mice against challenge with serotype Ia/c GBS strain C388/90.**

| Treatment of dams (n)¹ | Survival in pups (%)² |
|---|---|
| 6F3 (5) | 3/52 (6) |
| 11D2 (2) | 3/14 (21) |
| 6F3-11D2 (2) | 15/23 (65) |
| Rabbit anti-Sip serum (4)' | 37/38 (97) |
| Irrelevant Mab (6) | 0/69 (0) |

| | |
|---|---|
| ¹ A maximum volume of 500 µl of semi-purified antibodies were administered i.v. to pregnant mice on day 16 of gestation. When a combination of two Mabs was passively administered to the pregnant mice, 250 µl of each Mab were pooled together before injection. ² Number of survivors was followed for 7 days after challenge. The pups were challenged s.c. with 50 µl containing 3-4x10⁴ cfu from the serotype Ia/c GBS strain C388/90 between 24 to 48 h after birth. | |

## Claims

1. An isolated polynucleotide encoding a polypeptide consisting of an amino acid sequence at least 85% identical over the full length of the amino acid sequence chosen from: SEQ ID NOS: 4, 6, 8, and 12 wherein the encoded polypeptide retains the ability to raise antibodies having binding specificity for Group B Streptococcus.

2. The isolated polynucleotide of Claim 1 wherein the encoded polypeptide consists of an amino acid sequence at least 90% identical over the full length of the amino acid sequence chosen from SEQ ID NOS: 4, 6, 8, and 12.

3. The isolated polynucleotide of Claim 1 wherein the encoded polypeptide consists of an amino acid sequence at least 95% identical over the full length of the amino acid sequence chosen from SEQ ID NOS: 4, 6, 8, and 12.

4. The isolated polynucleotide of Claim I wherein the encoded polypeptide consists of an amino acid sequence at least 99% identical over the full length of the amino acid sequence chosen from SEQ ID NOS: 4, 6, 8, and 12.

5. The isolated polynucleotide of Claim 1 wherein the encoded polypeptide consists of the amino acid sequence chosen from SEQ ID NOS: 4, 6, 8, and 12.

6. The isolated polynucleotide of Claim 1 consisting of a nucleotide sequence chosen from: SEQ ID NOS: 3, 5, 7, and 11.

7. An isolated polynucleotide that is complementary to the isolated polynucleotide of any one of Claims 1 to 6.

8. A vector comprising the polynucleotide of any one of Claims 1 to 6, wherein said polynucleotide is operably linked to an expression control region.

9. A host cell transfected with the vector of Claim 8.

10. A process for producing a polypeptide comprising culturing the host cell according to Claim 9 under conditions suitable for expression of said polypeptide.

11. An isolated polypeptide consisting of an amino acid sequence at least 85% identical over the full length of the amino acid sequence chosen from: SEQ ID NOS: 4, 6, 8, and 12, wherein the polypeptide retains the ability to raise antibodies having binding specificity for Group B Streptococcus.

12. The polypeptide of Claim 11 consisting of an amino acid sequence at least 90% identical over the full length of the amino acid sequence chosen from SEQ ID NOS: 4, 6, 8, and 12.

13. The polypeptide of Claim 11 consisting of an amino acid sequence at least 95% identical over the full length of the amino acid sequence chosen from SEQ ID NOS: 4, 6, 8, and 12.

14. The isolated polypeptide of Claim 11 consisting of an amino acid sequence at least 99% identical over the full length of the amino acid sequence chosen from SEQ ID NOS: 4, 6, 8, and 12.

15. The isolated polypeptide of Claim 11 consisting of the amino acid sequence chosen from SEQ ID NOS:4, 6, 8, and 12.

16. A fusion polypeptide comprising the isolated polypeptide of any one of claims 11-15.

17. A chimeric polypeptide comprising two or more polypeptides wherein each of the two or more polypeptides consists of an amino acid sequence chosen from SEQ ID NOS:4, 6, 8, and 12, provided that the two or more polypeptides are linked as to form a chimeric polypeptide.

18. A vaccine comprising the polypeptide of any one of claims 11 to 15 and a pharmaceutically acceptable carrier, diluent or adjuvant.

19. A vaccine comprising the fusion polypeptide according to claim 16 or the chimeric polypeptide of claim 17 and a pharmaceutically acceptable carrier, diluent or adjuvant.

20. A method for detection of an antibody specific to Group B Streptococcus antigen in a biological sample previously obtained from a host, comprising:
(a) incubating one or more of the polypeptides according to any one of Claims 11 to 16 with the biological sample to form a mixture; and
(b) detecting specifically bound polypeptide in the mixture which indicates the presence of an antibody specific to the Group B Streptococcus antigen in the sample.

21. The vaccine according to any of Claims 18 to 19 for the prophylactic or therapeutic treatment of Group B Streptococcus infection in a host susceptible to Group B Streptococcus infection.

22. The vaccine according to any of Claims 18 to 19 for the prophylactic or therapeutic treatment of sepsis, meningitis, pneumonia, cellulitis, osteomyelitis, septic arthritis, endocarditis, or epiglottitis in a host.

23. The polypeptide according to any one of Claims 11 to 15 for prophylactic or therapeutic treatment of Group B Streptococcus infection in a host susceptible to Group B Streptococcus infection.

24. The polypeptide according to any one of Claims 11 to 15 for prophylactic or therapeutic treatment of sepsis, meningitis, pneumonia, cellulitis, osteomyelitis, septic arthritis, endocarditis, or epiglottitis in a host.

25. The vaccine of Claim 21 or Claim 22 or the polypeptide of Claim 23 or Claim 24 wherein the host is an animal.

26. The vaccine of Claim 21 or Claim 22 or the polypeptide of Claim 23 or Claim 24 wherein the host is chosen from a dairy herd.

27. The vaccine of Claim 21 or Claim 22 or the polypeptide of Claim 23 or Claim 24 wherein the host is a human.

28. Use of the polypeptide according to any one of Claims 11 to 15 in the manufacture of a medicament for the prophylactic or therapeutic treatment of sepsis, meningitis, pneumonia, cellulitis, osteomyelitis, septic arthritis, endocarditis, or epiglottitis.

29. Use of the polypeptide according to any one of Claims 11 to 15 in the manufacture of a medicament for the prophylactic or therapeutic treatment of Group B Streptococcus infection in a host susceptible to Group B Streptococcus infection.

30. A kit comprising the polypeptide according to any one of Claims 11 to 15 for detection or diagnosis of Group B Streptococcus infection.

## Patentansprüche

1. Isoliertes Polynukleotid, das ein Polypeptid kodiert, das aus einer Aminosäuresequenz mit mindestens 85 % Identität über die gesamte Länge der Aminosäuresequenz ausgewählt aus den SEQ ID Nrn.: 4, 6, 8 und 12 besteht, wobei das kodierte Polypeptid die Fähigkeit behält, Antikörper mit Bindungsspezifität für Gruppe B Streptococcus zu erzeugen.

2. Isoliertes Polynukleotid gemäß Anspruch 1, wobei das kodierte Polypeptid aus einer Aminosäuresequenz mit mindestens 90 % Identität über die gesamte Länge der Aminosäuresequenz ausgewählt aus den SEQ ID Nrn.: 4, 6, 8 und 12 besteht.

3. Isoliertes Polynukleotid gemäß Anspruch 1, wobei das kodierte Polypeptid aus einer Aminosäuresequenz mit mindestens 95 % Identität über die gesamte Länge der Aminosäuresequenz ausgewählt aus den SEQ ID Nrn.: 4, 6, 8 und 12 besteht.

4. Isoliertes Polynukleotid gemäß Anspruch 1, wobei das kodierte Polypeptid aus einer Aminosäuresequenz mit mindestens 99 % Identität über die gesamte Länge der Aminosäuresequenz ausgewählt aus den SEQ ID Nrn.: 4, 6, 8 und 12 besteht.

5. Isoliertes Polynukleotid gemäß Anspruch 1, wobei das kodierte Polypeptid aus der Aminosäuresequenz ausgewählt aus den SEQ ID Nrn.: 4, 6, 8 und 12 besteht.

6. Isoliertes Polynukleotid gemäß Anspruch 1 bestehend aus einer Nukleotidsequenz ausgewählt aus den SEQ ID Nrn.: 3, 5, 7 und 11.

7. Isoliertes Polynukleotid, das komplementär zu dem isolierten Polynukleotid gemäß einem der Ansprüche 1 bis 6 ist.

8. Vektor, umfassend das Polynukleotid gemäß einem der Ansprüche 1 bis 6, wobei das Polynukleotid funktionell mit einer Expressions-Kontrollregion verknüpft ist.

9. Wirtszelle, die mit dem Vektor gemäß Anspruch 8 transfiziert ist.

10. Verfahren zur Herstellung eines Polypeptids, umfassend die Kultivierung der Wirtszelle gemäß Anspruch 9 unter Bedingungen, die für die Expression des Polypeptids geeignet sind.

11. Isoliertes Polypeptid, bestehend aus einer Aminosäuresequenz mit mindestens 85 % Identität über die gesamte Länge der Aminosäuresequenz ausgewählt aus den SEQ ID Nrn.: 4, 6, 8 und 12, wobei das Polypeptid die Fähigkeit behält, Antikörper mit Bindungsspezifität für Gruppe B Streptococcus zu erzeugen.

12. Polypeptid gemäß Anspruch 11, bestehend aus einer Aminosäuresequenz mit mindestens 90 % Identität über die gesamte Länge der Aminosäuresequenz ausgewählt aus den SEQ ID Nrn.: 4, 6, 8 und 12.

13. Polypeptid gemäß Anspruch 11, bestehend aus einer Aminosäuresequenz mit mindestens 95 % Identität über die gesamte Länge der Aminosäuresequenz ausgewählt aus den SEQ ID Nrn.: 4, 6, 8 und 12.

14. Isoliertes Polypeptid gemäß Anspruch 11, bestehend aus einer Aminosäuresequenz mit mindestens 99 % Identität über die gesamte Länge der Aminosäuresequenz ausgewählt aus den SEQ ID Nrn.: 4, 6, 8 und 12.

15. Isoliertes Polypeptid gemäß Anspruch 11, bestehend aus der Aminosäuresequenz ausgewählt aus den SEQ ID Nrn.: 4, 6, 8 und 12.

16. Fusionspolypeptid, umfassend das isolierte Polypeptid gemäß einem der Ansprüche 11 bis 15.

17. Chimäres Polypeptid, umfassend zwei oder mehr Polypeptide, wobei jedes der zwei oder mehr Polypeptide aus einer Aminosäuresequenz, ausgewählt aus den SEQ ID Nrn.: 4, 6, 8 und 12 besteht, vorausgesetzt, dass die zwei oder mehr Polypeptide so verknüpft sind, dass sie ein chimäres Polypeptid bilden.

18. Impfstoff, umfassend das Polypeptid gemäß einem der Ansprüche 11 bis 15 und ein(en) pharmazeutisch akzeptable(n/s) Träger, Verdünnungsmittel oder Adjuvans.

19. Impfstoff, umfassend das Fusionspolypeptid gemäß Anspruch 16 oder das chimäre Polypeptid gemäß Anspruch 17 und ein(en) pharmazeutisch akzeptable(n/s) Träger, Verdünnungsmittel oder Adjuvans.

20. Verfahren zum Nachweis eines Antikörpers, der spezifisch für Gruppe B-Streptococcus-Antigen ist, in einer biologischen Probe, die vorher aus einem Wirt gewonnen wurde, umfassend:
(a) Inkubation eines oder mehrerer der Polypeptide gemäß einem der Ansprüche 11 bis 16 mit der biologischen Probe, um eine Mischung zu bilden; und
(b) Nachweis von spezifisch gebundenem Polypeptid in der Mischung, das die Anwesenheit eines Antikörpers in der Probe anzeigt, der spezifisch für das Gruppe B-Streptococcus-Antigen ist.

21. Impfstoff gemäß einem der Ansprüche 18 bis 19 für die prophylaktische oder therapeutische Behandlung einer Gruppe B-Streptococcus-Infektion in einem Wirt, der für eine Gruppe B-Streptococcus-Infektion anfällig ist.

22. Impfstoff gemäß einem der Ansprüche 18 bis 19 für die prophylaktische oder therapeutische Behandlung von Sepsis, Meningitis, Pneumonie, Cellulitis, Osteomyelitis, septischer Arthritis, Endokarditis oder Epiglottitis in einem Wirt.

23. Polypeptid gemäß einem der Ansprüche 11 bis 15 für die prophylaktische oder therapeutische Behandlung einer Gruppe B-Streptococcus-Infektion in einem Wirt, der für eine Gruppe B-Streptococcus-Infektion anfällig ist.

24. Polypeptid gemäß einem der Ansprüche 11 bis 15 für die prophylaktische oder therapeutische Behandlung von Sepsis, Meningitis, Pneumonie, Cellulitis, Osteomyelitis, septischer Arthritis, Endokarditis oder Epiglottitis in einem Wirt.

25. Impfstoff gemäß Anspruch 21 oder 22 oder Polypeptid gemäß Anspruch 23 oder 24, wobei der Wirt ein Tier ist.

26. Impfstoff gemäß Anspruch 21 oder 22 oder Polypeptid gemäß Anspruch 23 oder 24, wobei der Wirt aus einer Milchviehherde ausgewählt ist.

27. Impfstoff gemäß Anspruch 21 oder 22 oder Polypeptid gemäß Anspruch 23 oder 24, wobei der Wirt ein Mensch ist.

28. Verwendung des Polypeptids gemäß einem der Ansprüche 11 bis 15 zur Herstellung eines Medikaments für die prophylaktische oder therapeutische Behandlung von Sepsis, Meningitis, Pneumonie, Cellulitis, Osteomyelitis, septischer Arthritis, Endokarditis oder Epiglottitis.

29. Verwendung des Polypeptids gemäß einem der Ansprüche 11 bis 15 zur Herstellung eines Medikaments für die prophylaktische oder therapeutische Behandlung einer Gruppe B-Streptococcus-Infektion in einem Wirt, der für eine Gruppe B-Streptococcus-Infektion anfällig ist.

30. Kit, umfassend das Polypeptid gemäß einem der Ansprüche 11 bis 15, für den Nachweis oder die Diagnose einer Gruppe B-Streptococcus-Infektion.

## Revendications

1. Polynucléotide isolé codant pour un polypeptide consistant en une séquence d'acides aminés identique à au moins 85 % sur la longueur totale de la séquence d'acides aminés choisie parmi : SEQ ID N° : 4, 6, 8 et 12, le polypeptide codé conservant la capacité à diriger des anticorps ayant une spécificité de liaison pour *Streptococcus* de groupe B.

2. Polynucléotide isolé selon la revendication 1, le polypeptide codé consistant en une séquence d'acides aminés identique à au moins 90 % sur la longueur totale de la séquence d'acides aminés choisie parmi SEQ ID N° : 4, 6, 8 et 12.

3. Polynucléotide isolé selon la revendication 1, le polypeptide codé consistant en une séquence d'acides aminés identique à au moins 95 % sur la longueur totale de la séquence d'acides aminés choisie parmi SEQ ID N° : 4, 6, 8 et 12.

4. Polynucléotide isolé selon la revendication 1, le polypeptide codé consistant en une séquence d'acides aminés identique à au moins 99 % sur la longueur totale de la séquence d'acides aminés choisie parmi SEQ ID N° : 4, 6, 8 et 12.

5. Polynucléotide isolé selon la revendication 1, le polypeptide codé consistant en la séquence d'acides aminés choisie parmi SEQ ID N° : 4, 6, 8 et 12.

6. Polynucléotide isolé selon la revendication 1, consistant en une séquence de nucléotides choisie parmi SEQ ID N° : 3, 5, 7 et 11.

7. Polynucléotide isolé qui est complémentaire au polynucléotide isolé selon l'une quelconque des revendications 1 à 6.

8. Vecteur comprenant le polynucléotide selon l'une quelconque des revendications 1 à 6, dans lequel ledit polynucléotide est lié fonctionnellement à une région de contrôle de l'expression.

9. Cellule hôte transfectée avec le vecteur de la revendication 8.

10. Procédé de production d'un polypeptide comprenant la culture de la cellule hôte selon la revendication 9 dans des conditions appropriées pour l'expression dudit polypeptide.

11. Polypeptide isolé consistant en une séquence d'acides aminés identique à au moins 85 % sur la longueur totale de la séquence d' acides aminés choisie parmi SEQ ID N° 4, 6, 8 et 12, le polypeptide conservant la capacité à diriger des anticorps ayant une spécificité de liaison pour *Streptococcus* de groupe B.

12. Polypeptide selon la revendication 11, consistant en une séquence d' acides aminés identique à au moins 90 % sur la longueur totale de la séquence d'acides aminés choisie parmi SEQ ID N° : 4, 6, 8 et 12.

13. Polypeptide selon la revendication 11, consistant en une séquence d' acides aminés identique à au moins 95 % sur la longueur totale de la séquence d'acides aminés choisie parmi SEQ ID N° 4, 6, 8 et 12.

14. Polypeptide isolé selon la revendication 11, consistant en une séquence d'acides aminés identique à au moins 99 % sur la longueur totale de la séquence d'acides aminés choisie parmi SEQ ID N° : 4, 6, 8 et 12.

15. Polypeptide isolé selon la revendication 11, consistant en la séquence d'acides aminés choisie parmi SEQ ID N° : 4, 6, 8 et 12.

16. Polypeptide de fusion comprenant le polypeptide isolé selon l'une des revendications 11 à 15.

17. Polypeptide chimérique comprenant deux polypeptides ou plus, chacun des deux polypeptides ou plus consistant en une séquence d'acides aminés choisie parmi SEQ N° 4, 6, 8 et 12, à condition que les deux polypeptides ou plus soient liés de façon à former un polypeptide chimérique.

18. Vaccin comprenant le polypeptide selon l'une quelconque des revendications 11 à 15 et véhicule, diluant ou adjuvant pharmaceutiquement acceptable.

19. Vaccin comprenant le polypeptide de fusion selon la revendication 16 ou polypeptide chimérique selon la revendication 17 et véhicule, diluant ou adjuvant pharmaceutiquement acceptable.

20. Procédé de détection d'un anticorps spécifique de l'antigène de *Streptococcus* de groupe B dans un échantillon biologique obtenu au préalable chez un hôte, comprenant :
(a) l'incubation d'un ou plusieurs des polypeptides selon l'une quelconque des revendications 11 à 16 avec l'échantillon biologique pour former un mélange ; et
(b) la détection du polypeptide lié spécifiquement dans le mélange, ce qui indique la présence d'un anticorps spécifique de l'antigène de *Streptococcus* du groupe B dans l'échantillon.

21. Vaccin selon l'une quelconque des revendications 18 à 19, pour le traitement prophylactique ou thérapeutique d'une infection à *Streptococcus* de groupe B chez un hôte sensible à une infection à *Streptococcus* de groupe B.

22. Vaccin selon l'une quelconque des revendications 18 à 19, pour le traitement prophylactique ou thérapeutique de la septicémie, de la méningite, de la pneumonie, de la cellulite, de l'ostéomyélite, de l'arthrite purulente, de l'endocardite ou de l'épiglottite chez un hôte.

23. Polypeptide selon l'une quelconque des revendications 11 à 15, pour le traitement prophylactique ou thérapeutique d'une infection à *Streptococcus* de groupe B chez un hôte sensible à une infection à *Stretococcus* du groupe B.

24. Polypeptide selon l'une quelconque des revendications 11 à 15, pour le traitement prophylactique ou thérapeutique de la septicémie, de la méningite, de la pneumonie, de la cellulite, de l'ostéomyélite, de l'arthrite purulente, de l'endocardite ou de l'épiglottite chez un hôte.

25. Vaccin selon la revendication 21 ou la revendication 22 ou polypeptide selon la revendication 23 ou la revendication 24, l'hôte étant un animal.

26. Vaccin selon la revendication 21 ou la revendication 22 ou polypeptide selon la revendication 23 ou la revendication 24, l'hôte étant choisi dans un troupeau de vaches laitières.

27. Vaccin selon la revendication 21 ou la revendication 22 ou polypeptide selon la revendication 23 ou la revendication 24, l'hôte étant un être humain.

28. Utilisation du polypeptide selon l'une quelconque des revendications 11 à 15, dans la production d'un médicament pour le traitement prophylactique ou thérapeutique de la septicémie, de la méningite, de la pneumonie, de la cellulite, de l'ostéomyélite, de l'arthrite purulente, de l'endocardite ou de l'épiglottite.

29. Utilisation du polypeptide selon l'une quelconque des revendications 11 à 15 dans la production d'un médicament pour le traitement prophylactique ou thérapeutique d'une infection à *Streptococcus* de groupe B chez un hôte sensible à une infection à *Streptococcus* de groupe B.

30. Kit comprenant le polypeptide selon l'une quelconque des revendications 11 à 15, pour la détection ou le diagnostic d'une infection à *Streptococcus* de groupe B.
